# EUROPEAN PATENT SPECIFICATION

(11) **EP 3 691 017 B1**
(45) Date of publication and mention of the grant of the patent: **12.03.2025**
(21) Application number: 20154278.4
(22) Date of filing: 29.01.2020
(51) Int. Cl.: H01M 10/0567, H01M 10/0525, H01M 4/525, H01M 4/02, H01M 4/36, H01M 4/38, H01M 4/587, H01M 10/0568, H01M 10/0569

(54) **LITHIUM SECONDARY BATTERY**
LITHIUMSEKUNDÄRBATTERIE
BATTERIE SECONDAIRE AU LITHIUM

(30) Priority: 01.02.2019 KR 20190013754
(43) Date of publication of application: 05.08.2020
(73) Proprietor: Samsung SDI Co., Ltd., Gyeonggi-do 17084 (KR); Samsung Electronics Co., Ltd., Gyeonggi-do 16677 (KR)
(72) Inventor: PARK, Insun, Suwon-si, Gyeonggi-do 16678 (KR); KOH, Myongchun, Suwon-si, Gyeonggi-do 16678 (KR); KIM, Dongyoung, Yongin-si, Gyeonggi-do 17084 (KR); SEO, Jinah, Yongin-si, Gyeonggi-do 17084 (KR)
(74) Representative: Elkington and Fife LLP

(56) References cited:
- EP-A1- 3 416 227
- US-A1- 2018 026 305
- US-A1- 2018 053 967

## Description

### FIELD OF THE INVENTION

The present disclosure relates to lithium batteries.

### BACKGROUND OF THE INVENTION

Lithium batteries are used as driving power sources for portable electronic devices including video cameras, mobile phones, notebook computers, and the like. Lithium secondary batteries, which are rechargeable, have an energy density per unit weight that is at least three times higher than that of existing lead storage batteries, nickel-cadmium batteries, nickel-hydrogen batteries, nickel-zinc batteries, or the like, and are able to be charged at high rates.

To manufacture lithium secondary batteries having high energy density, positive electrode active materials exhibiting enhanced discharge capacity are used. These positive electrode active materials have low electrochemical stability. Thus, a side reaction between a positive electrode active material and an electrolyte occurs during charging and discharging of a lithium secondary battery, resulting in deteriorated stability of the lithium secondary battery. Therefore, there is a need for a method of enhancing stability of a lithium secondary battery including a positive electrode active material exhibiting enhanced discharge capacity.

EP 3 416 227 A1 and US 2018/053967 A1 describe lithium secondary batteries including a positive electrode, a negative electrode and an electrolyte comprising a phosphate or phosphite compound as additive, disposed between said electrodes, wherein the positive electrode comprises a nickel-rich lithium composite oxide, such as LiNi _{0.88}Co_{0.08}Mn_{0.04}O₂ or LiNi_{0.88}Co_{0.08}Al_{0.04}O₂, as active material.

US 2018/026305 A1 discloses the use of alkyl sulfonate compounds as additive for the electrolytic solution of a lithium secondary battery comprising a lithium transition metal oxide as positive electrode active material.

### SUMMARY OF THE INVENTION

Provided are novel lithium batteries.

Additional aspects will be set forth in part in the description which follows and, in part, will be apparent from the description, or may be learned by practice of the presented embodiments.

According to the present invention, a lithium battery includes: a positive electrode; a negative electrode; and an electrolyte between the positive electrode and the negative electrode, wherein the positive electrode includes a positive electrode active material represented by Formula 1A below, and
the electrolyte includes a lithium salt, a non-aqueous solvent, and an alkyl sulfonate compound represented by Formula 2 below:

   <Formula 1A> LiₓNi_{y}M_{1-y}O_{2-z}A_{z}
wherein, in Formula 1A, 0.9≤x≤1.2, 0.88≤y≤0.98, and 0≤z<0.2 are satisfied,
M is at least one element selected from the group consisting of aluminum (Al), magnesium (Mg), manganese (Mn), cobalt (Co), iron (Fe), chromium (Cr), vanadium (V), titanium (Ti), copper (Cu), boron (B), calcium (Ca), zinc (Zn), zirconium (Zr), niobium (Nb), molybdenum (Mo), strontium (Sr), antimony (Sb), tungsten (W), and bismuth (Bi); and
A is an element having an oxidation number of -1 or -2; 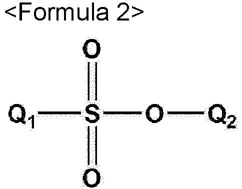
wherein, in Formula 2, Q₁ and Q₂ are each independently a substituted or unsubstituted C₁-C₂₀ alkyl group,
wherein the amount of the alkyl sulfonate compound is in a range of 0.005 parts by weight to 5 parts by weight with respect to 100 parts by weight of the electrolyte.
According to a preferred embodiment, the electrolyte includes:
a lithium salt;
a non-aqueous solvent;
an alkyl sulfonate compound represented by Formula 2 below; and
an unsaturated sulfone compound represented by Formula 3 below: 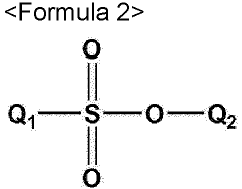
wherein, in Formula 2, Q₁ and Q₂ are each independently a substituted or unsubstituted C₁-C₂₀ alkyl group, and 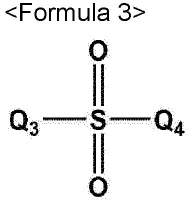
wherein, in Formula 3, Q₃ and Q₄ are each independently selected from a group represented by -(L₁)-(R₁), a vinyl group, an allyl group, and a substituted or unsubstituted C₁-C₂₀ alkyl group,
at least one of Q₃ and Q₄ is a group represented by -(L₁)-(R₁), a vinyl group, or an allyl group,
L₁ is selected from a substituted or unsubstituted C₂-C₂₀ alkenylene group and a substituted or unsubstituted C₂-C₂₀ alkynylene group, and
R₁ is selected from hydrogen and a substituted or unsubstituted C₂-C₂₀ alkyl group.

According to an aspect of another embodiment, a lithium battery includes: a positive electrode; a negative electrode; and an electrolyte between the positive electrode and the negative electrode, the electrolyte including a lithium salt, a non-aqueous solvent, the alkyl sulfonate compound, and the unsaturated sulfone compound,
wherein the positive electrode comprises a positive electrode active material represented by Formula 1B below:

   <Formula 1B> LiₚNi_{q}M_{1-q}O₂₋ᵣAᵣ
wherein, in Formula 1B, 0.9≤p≤1.2, 0.7≤q≤0.98, and 0≤r<0.2 are satisfied;
M is at least one element selected from the group consisting of aluminum (Al), magnesium (Mg), manganese (Mn), cobalt (Co), iron (Fe), chromium (Cr), vanadium (V), titanium (Ti), copper (Cu), boron (B), calcium (Ca), zinc (Zn), zirconium (Zr), niobium (Nb), molybdenum (Mo), strontium (Sr), antimony (Sb), tungsten (W), and bismuth (Bi); and
A is an element having an oxidation number of -1, -2, or -3.

### BRIEF DESCRIPTION OF THE DRAWINGS

These and/or other aspects will become apparent and more readily appreciated from the following description of the embodiments, taken in conjunction with the accompanying drawing in which:

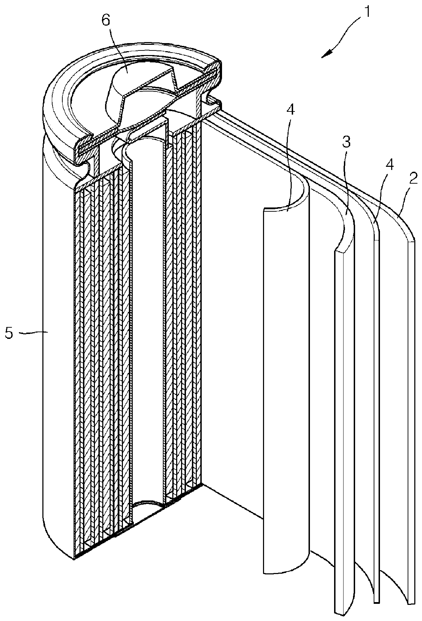

FIG. 1 is a view of a lithium battery according to an example embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

Hereinafter, a lithium battery according to example embodiments, an electrolyte for a lithium battery, and a lithium battery employing the electrolyte will be described in more detail.

A lithium battery according to an embodiment includes a positive electrode, a negative electrode, and an electrolyte between the positive electrode and a negative electrode,
wherein the positive electrode includes a positive electrode active material represented by Formula 1A below, and
the electrolyte includes a lithium salt, a non-aqueous solvent, and an alkyl sulfonate compound represented by Formula 2 below:

   <Formula 1A> LiₓNi_{y}M_{1-y}O_{2-z}A_{z}

   wherein, in Formula 1A, 0.9≤x≤1.2, 0.88≤y≤0.98, and 0≤z<0.2 are satisfied,
   M is at least one element selected from the group consisting of aluminum (Al), magnesium (Mg), manganese (Mn), cobalt (Co), iron (Fe), chromium (Cr), vanadium (V), titanium (Ti), copper (Cu), boron (B), calcium (Ca), zinc (Zn), zirconium (Zr), niobium (Nb), molybdenum (Mo), strontium (Sr), antimony (Sb), tungsten (W), and bismuth (Bi); and
A is an element having an oxidation number of -1 or -2; 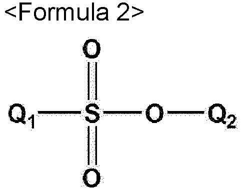
   wherein, in Formula 2, Q₁ and Q₂ are each independently a substituted or unsubstituted C₁-C₂₀ alkyl group,
   wherein the amount of the alkyl sulfonate compound is in a range of 0.005 parts by weight to 5 parts by weight with respect to 100 parts by weight of the electrolyte.

When a lithium metal composite oxide having a high Ni content is used, such as the positive electrode active material of Formula 1A, a high-output and high-capacity battery may be manufactured. However, Ni cations included in the lithium metal composite oxide are eluted from a positive electrode into an electrolyte, or Li on a surface of the positive electrode reacts with oxygen or the like, thus deteriorating the positive electrode, and these cations react with a solid electrolyte interface (SEI) film of a negative electrode, thereby degrading the SEI film such that a negative electrode active material is partially exposed to the electrolyte, and consequently, a side reaction occurs, resulting in deteriorated capacity and lifespan characteristics and an increase in the amount of gas generated.

To address these, the lithium battery includes an electrolyte including the alkyl sulfonate compound of Formula 2, and thus a side reaction by Ni cations is minimized and consequently, gas generation is reduced, resulting in enhanced battery lifespan.

In particular, the alkyl sulfonate compound has a high affinity with Ni cations, and thus has an effect of suppressing a side reaction with Ni cations, and particularly maintains a high affinity with Ni cations even while a battery is operated at a high voltage, thus having an effect of inhibiting the degradation of an SEI film.

In addition, the alkyl sulfonate compound may be adsorbed onto a surface of a positive electrode by interaction with Li cations, and thus may protect the surface of the positive electrode from oxygen, or the like. Thus, the alkyl sulfonate compound may reduce gas generation due to a side reaction at the surface of the positive electrode and lower battery resistance, thereby enhancing battery performance and lifespan.

In this regard, the amount of the alkyl sulfonate compound in the electrolyte is in a range of 0.005 parts by weight to 5 parts by weight with respect to 100 parts by weight of the electrolyte. That is, the amount of the alkyl sulfonate compound is within a range that enables stabilization of Ni cations eluted into an electrolyte from a positive electrode active material and protection of the surface of the positive electrode by interaction with Li cations in the electrolyte. When the amount of the alkyl sulfonate compound exceeds 5 parts by weight, the alkyl sulfonate compound is decomposed, and thus film resistance is increased, and produced CO₂ has an adverse effect, and accordingly, battery capacity, storage stability, and cycle characteristics may be deteriorated.

According to the present invention, the amount of the alkyl sulfonate compound is in a range of about 0.005 parts by weight to about 5 parts by weight with respect to 100 parts by weight of the electrolyte. For example, the amount of the alkyl sulfonate compound may be in a range of about 0.01 parts by weight to about 2 parts by weight, or about 0.1 parts by weight to about 1.5 parts by weight, with respect to 100 parts by weight of the electrolyte.

When the amount of the alkyl sulfonate compound is less than 0.005 parts by weight with respect to 100 parts by weight of the electrolyte, the amount is excessively small, such that it may be difficult to sufficiently obtain an effect of protecting a positive electrode.

According to one embodiment, in Formula 2, Q₁ and Q₂ may be each independently selected from: a methyl group, an ethyl group, a propyl group, an isopropyl group, a butyl group, a sec-butyl group, a tert-butyl group, and an isobutyl group; and
a methyl group, an ethyl group, a propyl group, an isopropyl group, a butyl group, a sec-butyl group, a tert-butyl group, or an isobutyl group that is substituted with at least one selected from deuterium, -F, -Cl ,-Br, -I, a cyano group, a nitro group, a hydroxyl group, a methyl group, an ethyl group, and a propyl group.

For example, the alkyl sulfonate compound may be at least one selected from compounds 1 and 2 below:

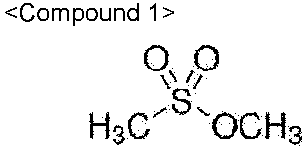

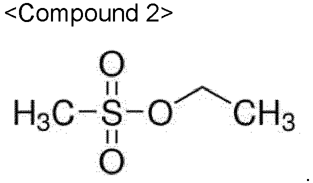

The alkyl sulfonate compound of Formula 2 reduces gas generation of a lithium battery due to a high oxidation voltage thereof, may protect the surface of the positive electrode from oxygen by being adsorbed onto the surface of the positive electrode during charging, and suppresses the degradation of an SEI film by stabilizing Ni cations, and consequently, gas generation from a side reaction at the surface of the positive electrode is reduced and battery resistance is lowered, and accordingly, a lithium battery may exhibit enhanced performance and lifespan characteristics.

The electrolyte includes a lithium salt. The lithium salt may act as a supply source of lithium ions in a battery by being dissolved in an organic solvent, and for example, may serve to promote the transfer of lithium ions between a positive electrode and a negative electrode.

An anion of the lithium salt included in the electrolyte may be at least one selected from the group consisting of PF₆⁻, BF₄⁻, SbF₆⁻, AsF₆⁻, C₄F₉SO₃⁻, ClO₄⁻, AlO₂⁻, AlCl₄⁻, CₓF₂ₓ₊₁SO₃⁻ (wherein, x is a natural number, for example, 1 to 10), (CₓF₂ₓ₊₁SO₂)(C_{y}F_{2y+1}SO₂)N⁻ (wherein, x and y are natural numbers, for example, independently 1 to 10), and a halide.

In the electrolyte, the lithium salt may include at least one selected from LiPF₆, LiBF₄, LiCF₃SO₃, Li(CF₃SO₂)₂N, LiC₂F₅SO₃, Li(FSO₂)₂N, LiC₄F₉SO₃, LiN(SO₂CF₂CF₃)₂, and compounds represented by Formulae 22 to 25 below:

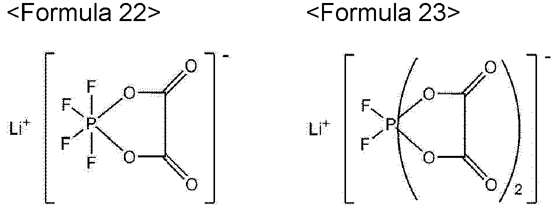

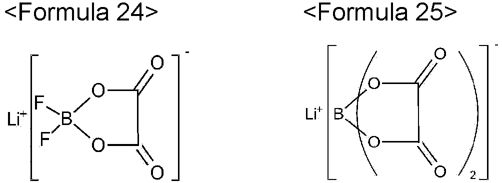

The concentration of the lithium salt may be in a range of about 0.01 M to about 5.0 M, about 0.05 M to about 5.0 M, about 0.1 M to about 5.0 M, or about 0.1 M to about 2.0 M, but the present disclosure is not particularly limited thereto, and as needed, an appropriate concentration of the lithium salt may be used.

The amount of the lithium salt in a solvent-free electrolyte may be in a range of about 0.001 parts by weight to about 30 parts by weight with respect to 100 parts by weight of the solvent-free electrolyte, but the present disclosure is not particularly limited thereto, and the lithium salt may be used within any amount range that enables the electrolyte to effectively transfer lithium ions and/or electrons during charging and discharging processes.

The amount of the lithium salt in a solvent-containing electrolyte may be in a range of about 100 mM to about 10 M. For example, the amount of the lithium salt may be in a range of about 100 mM to about 2 M. For example, the amount of the lithium salt may be in a range of about 500 mM to about 2 M. However, the present disclosure is not particularly limited thereto and the lithium salt may be used within any amount range that enables the electrolyte to effectively transfer lithium ions and/or electrons during charging and discharging processes.

According to one embodiment, the concentration of the lithium salt in the electrolyte may be in a range of about 1.1 M to about 2.5 M. For example, the concentration of the lithium salt may be in a range of about 1.15 M to about 2.2 M, or about 1.3 M to about 2 M.

The non-aqueous solvent may be selected from the group consisting of a carbonate-based solvent, an ester-based solvent, an ether-based solvent, a ketone-based solvent, an aprotic solvent, and a mixture thereof.

Non-limiting examples of the carbonate-based solvent include dimethyl carbonate (DMC), diethyl carbonate (DEC), ethyl methyl carbonate (EMC), dipropyl carbonate (DPC), methyl propyl carbonate (MPC), ethyl propyl carbonate (EPC), methyl ethyl carbonate (MEC), ethylene carbonate (EC), propylene carbonate (PC), butylene carbonate (BC), and tetraethylene glycol dimethyl ether (TEGDME). Non-limiting examples of the ester-based solvent include methyl acetate, ethyl acetate, n-propyl acetate, dimethyl acetate, methyl propionate (MP), ethyl propionate, γ-butyrolactone, decanolide, valerolactone, mevalonolactone, and caprolactone. Non-limiting examples of the ether-based solvent include dibutyl ether, tetraglyme, diglyme, dimethoxyethane, 2-methyl tetrahydrofuran, and tetrahydrofuran, and the ketone-based solvent may be cyclohexanone or the like. As a nitrile-based solvent, acetonitrile (AN), succinonitrile (SN), adiponitrile, or the like may be used.

The aprotic solvents may be used alone or at least one of these solvents may be used in combination. In a case in which one or more of these solvents are mixed, a mixing ratio may be appropriately adjusted according to the desired battery performance, as obvious to one of ordinary skill in the art.

As other solvents, dimethylsulfoxide, dimethylformamide, dimethylacetamide, tetrahydrofuran, and the like may be used, but the present disclosure is not particularly limited thereto, and any organic solvent that may be used in the art is possible.

For example, the non-aqueous solvent may include about 50 vol% to about 95 vol% of a chain carbonate and about 5 vol% to about 50 vol% of a cyclic carbonate, about 55 vol% to about 95 vol% of a chain carbonate and about 5 vol% to about 45 vol% of a cyclic carbonate, about 60 vol% to about 95 vol% of a chain carbonate and about 5 vol% to about 40 vol% of a cyclic carbonate, about 65 vol% to about 95 vol% of a cyclic carbonate and about 5 vol% to about 35 vol% of a cyclic carbonate, or about 70 vol% to about 95 vol% of a chain carbonate and about 5 vol% to about 30 vol% of a cyclic carbonate. For example, the non-aqueous solvent may be a mixture of three or more non-aqueous solvents.

In some embodiments, the non-aqueous solvent may further include fluoro-ethylene carbonate (FEC), vinylene carbonate (VC), vinyl ethylene carbonate (VEC), a phosphorus (P)-containing compound, a sulfur (S)-containing compound, or the like.

For example, the non-aqueous solvent may include FEC. For example, the lithium battery may include the non-aqueous solvent in an amount of about 0.1 vol% to about 10 vol% with respect to a total volume of the non-aqueous solvent. For example, the lithium battery may include the non-aqueous solvent in an amount of about 0.5 vol% to about 7 vol% with respect to the total volume of the non-aqueous solvent. For example, the lithium battery may include the non-aqueous solvent in an amount of about 1 vol% to about 7 vol% with respect to the total volume of the non-aqueous solvent. For example, the lithium battery may include the non-aqueous solvent in an amount of about 2 vol% to about 7 vol% with respect to the total volume of the non-aqueous solvent. When the amount of the FEC in the non-aqueous solvent is within the above range, an effective SEI film that does not hinder a diffusion rate of lithium ions may be rapidly formed.

According to one embodiment, the non-aqueous solvent may be selected from dimethyl carbonate (DMC), diethyl carbonate (DEC), ethyl methyl carbonate (EMC), dipropyl carbonate (DPC), methyl propyl carbonate (MPC), ethyl propyl carbonate (EPC), methyl ethyl carbonate (MEC), ethylene carbonate (EC), propylene carbonate (PC), butylene carbonate (BC), methyl propionate (MP), ethyl propionate, propyl propionate (PP), tetraethylene glycol dimethyl ether (TEGDME), and mixtures thereof.

The electrolyte may include a carbonate containing a carbon-carbon single or multiple bond, a carboxylic acid anhydride containing a carbon-carbon single or multiple bond, or a mixture thereof. The multiple bond may be a double bond or a triple bond, and the carbonate and the carboxylic acid anhydride may be linear or cyclic.

According to one embodiment, the electrolyte may include a cyclic carbonate compound, a cyclic acid anhydride compound, a P-containing compound, an S-containing compound, or a mixture thereof.

According to one embodiment, the electrolyte includes a cyclic carbonate compound, a cyclic acid anhydride compound, or a mixture thereof, and the alkyl sulfonate compound may have a higher reduction potential than that of the cyclic carbonate compound or the cyclic acid anhydride compound.

In addition, according to one embodiment, the amount of the cyclic carbonate compound, the cyclic acid anhydride compound, or a mixture thereof may be in a range of about 0.1 parts by weight to about 2 parts by weight with respect to 100 parts by weight of the electrolyte.

The cyclic carbonate compound may be, for example, at least one selected from fluoro-ethylene carbonate (FEC), vinylene carbonate (VC), and vinyl ethylene carbonate (VEC).

The cyclic acid anhydride compound may be, for example, at least one selected from maleic anhydride and succinic anhydride.

The P-containing compound may be, for example, at least one selected from a phosphine compound, a phosphate compound, and a phosphite compound.

The phosphine compound may be particularly triphenylphosphine, tris(4-fluorophenyl)phosphine, tris(2,4-difluorophenyl)phosphine, or tris(perfluorophenyl)phosphine, but the present disclosure is not limited thereto. The phosphate compound may be particularly triphenyl phosphate (TPPa) or trimethyl phosphate (TMPa), but the present disclosure is not limited thereto. The phosphite compound may be particularly triethylphosphite (TEPi), trimethylphosphite, tripropylphosphite, tributylphosphite, tris(trimethylsillyl)phosphite, or triphenylphosphite, but the present disclosure is not limited thereto.

The S-containing compound may be, for example, at least one selected from a sulfone compound, a sulfonate compound, a sultone compound, and a disulfonate compound.

The sulfone compound may be particularly ethylmethyl sulfone, divinyl sulfone, or tetramethylene sulfone, but the present disclosure is not limited thereto. The sulfonate compound may be particularly methyl methane sulfonate, ethyl methane sulfonate, or diallyl sulfonate, but the present disclosure is not limited thereto. The disulfonate compound may be particularly methylene methane disulfonate (MMDS) or busulfan, but the present disclosure is not limited thereto. The sultone compound may be particularly fluoropropate sultone (FPS), but the present disclosure is not limited thereto.

According to one embodiment, the lithium battery may include the electrolyte in an amount of about 1 g/Ah to about 3 g/Ah.

The positive electrode includes the positive electrode active material of Formula 1A.

For example, in Formula 1A, A may be one of halogens, S, and nitrogen (N), but the present disclosure is not limited thereto.

In Formula 1A, y denotes the content of nickel (Ni) in the positive electrode active material. According to one embodiment, in Formula 1A, y may satisfy the following condition: 0.88≤y≤0.98.

In addition, according to one embodiment, in Formula 1A, M may be at least one element of Co, Ni, and Mn.

For example, when the positive electrode includes the positive electrode active material of Formula 1A, the positive electrode may include at least one selected from Li_{1.02}Ni_{0.88}Co_{0.08}Mn_{0.04}O₂, Li_{1.02}Ni_{0.88}Co_{0.10}Mn_{0.02}O₂, Li_{1.02}Ni_{0.91}Co_{0.06}Mn_{0.03}O₂, LiNi_{0.94}Co_{0.04}Mn_{0.02}O₂, Li_{1.02}Ni_{0.88}Co_{0.08}Al_{0.04}O₂, Li_{1.02}Ni_{0.88}Co_{0.10}Al_{0.02}O₂, Li_{1.02}Ni_{0.91}Co_{0.06}Al_{0.03}O₂, and LiNi_{0.94}Co_{0.04}Al_{0.02}O₂.

For example, the positive electrode active material may be represented by Formula 10 or 20:

<Formula 10> Li_{x'}Ni_{y'}Co_{n-y'-z'}Al_{z'}O₂

<Formula 20> Li_{x'}Ni_{y'}Co_{1-y'-z'}Mn_{z'}O₂

In Formulae 10 and 20, 0.9≤x'≤1.2, 0.88≤y'≤0.98, 0<z'<0.1, and 0<1-y'-z'<0.2 are satisfied.

As described above, in the case of a lithium metal oxide having a high Ni content, a high-capacity battery may be realized, but the content of Ni cations in batteries of the related art increases, resulting in deteriorated lifespan characteristics.

In addition, as described below, gas is generated in a lithium battery including a negative electrode active material including a metal alloyable with lithium or a carbon-based negative electrode active material due to catalytic action at a high temperature, and accordingly, the lithium battery exhibits deteriorated lifespan characteristics.

As described above, when the amount of FEC, VC, VEC, MA, SA, the P-containing compound, or the S-containing compound is within the above range, a passivation film including a chemical reaction product of the above material, i.e., an SEI film, may be formed on a part or all of a surface of the negative electrode.

In addition, the positive electrode may further include, in addition to the above-listed positive electrode active materials, at least one selected from the group consisting of lithium cobalt oxide, lithium nickel cobalt manganese oxide, lithium nickel cobalt aluminum oxide, lithium iron phosphate, and lithium manganese oxide, but the present disclosure is not particularly limited thereto, and the positive electrode may further include any positive electrode active material that may be used in the art.

The negative electrode includes a negative electrode active material, and the negative electrode active material may include at least one selected from a silicon-based compound, a carbon-based compound, a composite of a silicon-based compound and a carbon-based compound, and a silicon oxide (SiOₓ₁ where 0<x1<2). In another embodiment, for example, the negative electrode may include a negative electrode active material including a metal alloyable with lithium, a silicon-based negative electrode active material, and/or a carbon-based negative electrode active material.

For example, the silicon-based compound may include silicon particles having an average diameter of 200 nm or less.

For example, the carbon-based compound may include graphite.

For example, the composite of a silicon-based compound and a carbon-based compound may be a composite having a structure in which silicon nanoparticles are arranged on the carbon-based compound, a composite in which silicon particles are included on a surface of and inside the carbon-based compound, or a composite in which silicon particles are coated with the carbon-based compound and thereby included in the carbon-based compound. The composite of a silicon-based compound and a carbon-based compound may be an active material obtained by dispersing silicon nanoparticles having an average diameter of about 200 nm or less on carbon-based compound particles, and then coating the resulting structure with carbon, an active material in which silicon particles are present on and inside graphite, or the like. Secondary particles of the composite of a silicon-based compound and a carbon-based compound may have an average diameter of about 5 µm to about 20 µm, and silicon nanoparticles may have an average diameter of 200 nm or less, 150 nm or less, 100 nm or less, 50 nm or less, 20 nm or less, or 10 nm or less. For example, the average diameter of the silicon nanoparticles may be in a range of about 100 nm to about 150 nm.

For example, the composite of a silicon-based compound and a carbon-based compound may have a capacity of about 300 mAh/g to about 700 mAh/g. For example, the capacity of the composite of a silicon-based compound and a carbon-based compound may be in a range of about 400 mAh/g to about 600 mAh/g.

The lithium battery may have a capacity retention rate of 80% or higher, for example, 82% or higher, after 200 cycles of charging and discharging at 25 °C. For example, when the negative electrode of the lithium battery includes a silicon compound or a silicon oxide, the capacity retention rate of the lithium battery after 200 cycles of charging and discharging at 25 °C may be 85% or higher.

The lithium battery may have a direct current internal resistance (DCIR) increase rate of 150% or less after 200 cycles of charging and discharge at 25 °C. For example, when the negative electrode of the lithium battery includes a silicon compound or a silicon oxide, the DCIR increase rate of the lithium battery after 200 cycles of charging and discharge at 25 °C may be 135% or less, for example, 125% or less.

The lithium battery may have a DCIR increase rate of 100% or less after being stored at 60 °C for 10 days. That is, a lithium battery including the alkyl sulfonate compound in the electrolyte and having a Ni content of 88 wt% or higher in the positive electrode active material may exhibit a substantially reduced DCIR when stored at a high temperature.

The lithium battery may have a cell energy density per unit volume of 500 Wh/L or more. Since the lithium battery provides a high energy density, i.e., 500 Wh/L or higher, high output may be obtained.

According to a preferred embodiment, the electrolyte includes a lithium salt; a non-aqueous solvent;
an alkyl sulfonate compound represented by Formula 2 below; and
an unsaturated sulfone compound represented by Formula 3 below: 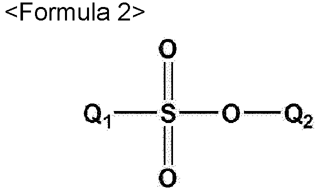
   wherein, in Formula 2, Q₁ and Q₂ are each independently a substituted or unsubstituted C₁-C₂₀ alkyl group, 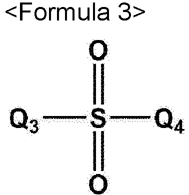
   wherein, in Formula 3, Q₃ and Q₄ are each independently selected from a group represented by -(L₁)-(R₁), a vinyl group, an allyl group, and a substituted or unsubstituted C₁-C₂₀ alkyl group,
   at least one of Q₃ and Q₄ is a group represented by -(L₁)-(R₁), a vinyl group, or an allyl group,
   L₁ is selected from a substituted or unsubstituted C₂-C₂₀ alkenylene group and a substituted or unsubstituted C₂-C₂₀ alkynylene group, and
   R₁ is selected from hydrogen and a substituted or unsubstituted C₂-C₂₀ alkyl group.

When a lithium metal composite oxide having a high Ni content, such as the positive electrode active material of Formula 1B is used, a high-output and high-capacity battery may be manufactured, but Ni cations included in the lithium metal composite oxide are eluted from a positive electrode into an electrolyte, or Li on a surface of the positive electrode reacts with oxygen or the like, thus deteriorating the positive electrode, and these cations react with an SEI film of a negative electrode, thereby degrading the SEI film, and accordingly, the negative electrode active material is partially exposed to the electrolyte, thus causing a side reaction or the like, resulting in deteriorated capacity and lifespan characteristics and an increase in the amount of gas generated.

To address these, the lithium battery includes an electrolyte including the alkyl sulfonate compound of Formula 2 and the unsaturated sulfone compound of Formula 3, and thus a side reaction by Ni cations is minimized and consequently, gas generation is reduced, resulting in enhanced battery lifespan.

In particular, the alkyl sulfonate compound has a high affinity with Ni cations, and thus has an effect of suppressing a side reaction with Ni cations, and particularly maintains a high affinity with Ni cations even while a battery is operated at a high voltage, thus having an effect of inhibiting the degradation of an SEI film.

In addition, the alkyl sulfonate compound may be adsorbed onto a surface of a positive electrode by interaction with Li cations, and thus may protect the surface of the positive electrode from oxygen, or the like. Thus, the alkyl sulfonate compound may reduce gas generation due to a side reaction at the surface of the positive electrode and lower battery resistance, thereby enhancing battery performance and lifespan.

In addition, the unsaturated sulfone compound may be reduced and decomposed at a metallic negative electrode before the solvent, thereby forming a more stable SEI film at the surface of the negative electrode. The SEI film formed on the surface of the negative electrode may reduce gas generation due to a side reaction, thereby enhancing electrochemical characteristics of a battery.

Thus, the electrolyte including the alkyl sulfonate compound and the unsaturated sulfone compound protects a positive electrode and enhances the stability of an SEI film, and thus gas generation of a lithium battery may be reduced and battery resistance may be lowered, resulting in enhanced battery performance and lifespan.

According to one embodiment, the amount of the alkyl sulfonate compound may be in a range of about 0.001 parts by weight to about 3 parts by weight with respect to 100 parts by weight of the electrolyte. For example, the amount of the alkyl sulfonate compound may be in a range of about 0.01 parts by weight to about 1.5 parts by weight, or about 0.1 parts by weight to about 0.5 parts by weight, with respect to 100 parts by weight of the electrolyte.

When the amount of the alkyl sulfonate compound is less than 0.001 parts by weight with respect to 100 parts by weight of the electrolyte, the amount is excessively small, such that it may be difficult to sufficiently obtain an effect of protecting a positive electrode.

According to one embodiment, the amount of the unsaturated sulfone compound may be in a range of about 0.001 parts by weight to about 3 parts by weight with respect to 100 parts by weight of the electrolyte. For example, the amount of the unsaturated sulfone compound may be in a range of about 0.01 parts by weight to about 1.5 parts by weight, or about 0.1 parts by weight to about 0.5 parts by weight, with respect to 100 parts by weight of the electrolyte.

When the amount of the unsaturated sulfone compound is less than 0.001 parts by weight with respect to 100 parts by weight of the electrolyte, the amount is excessively small, such that the SEI film may not be formed and it may be difficult to sufficiently obtain an effect of reducing resistance.

According to one embodiment, the sum of the amount of the alkyl sulfonate compound and the amount of the unsaturated sulfone compound may be in a range of about 0.001 parts by weight to about 5 parts by weight with respect to 100 parts by weight of the electrolyte. For example, the sum of the amount of the alkyl sulfonate compound and the amount of the unsaturated sulfone compound may be in a range of about 0.01 parts by weight to about 2 parts by weight, or about 0.1 parts by weight to about 1.5 parts by weight, with respect to 100 parts by weight of the electrolyte.

When the sum of the amount of the alkyl sulfonate compound and the amount of the unsaturated sulfone compound is less than 0.005 parts by weight with respect to 100 parts by weight of the electrolyte, the amount is excessively small, such that the SEI film may not be formed and it may be difficult to sufficiently obtain an effect of protecting a positive electrode and lowering resistance.

According to one embodiment, in Formula 2, Q₁ and Q₂ may be each independently selected from: a methyl group, an ethyl group, a propyl group, an isopropyl group, a butyl group, a sec-butyl group, a tert-butyl group, and an isobutyl group; and
a methyl group, an ethyl group, a propyl group, an isopropyl group, a butyl group, a sec-butyl group, a tert-butyl group, or an isobutyl group that is substituted with at least one selected from deuterium, -F, -Cl ,-Br, -I, a cyano group, a nitro group, a hydroxyl group, a methyl group, an ethyl group, and a propyl group.

According to one embodiment, in Formula 3, Q₃ and Q₄ may be each independently selected from: a vinyl group, an allyl group, a methyl group, an ethyl group, a propyl group, an isopropyl group, a butyl group, a sec-butyl group, a tert-butyl group, and an isobutyl group; and
a methyl group, an ethyl group, a propyl group, an isopropyl group, a butyl group, a sec-butyl group, a tert-butyl group, or an isobutyl group that is substituted with at least one selected from deuterium, -F, -Cl ,-Br, -I, a cyano group, a nitro group, a hydroxyl group, a methyl group, an ethyl group, and a propyl group, and
at least one of Q₃ and Q₄ may be a vinyl group or an allyl group.

For example, the alkyl sulfonate compound may be at least one selected from Compounds 1 and 2 below:

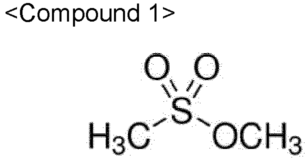

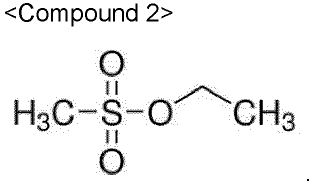

For example, the unsaturated sulfone compound may be Compound 3 below:

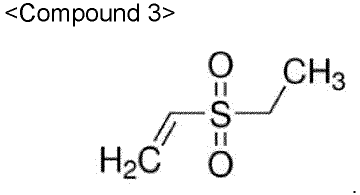

The alkyl sulfonate compound of Formula 2 may reduce gas generation of a lithium battery due to a high oxidation voltage thereof, protect the surface of the positive electrode from oxygen by being adsorbed onto the surface of the positive electrode during charging, and suppress the degradation of the SEI film by stabilizing Ni cations, thereby reducing gas generation due to a side reaction at the surface of the positive electrode and lowering battery resistance, and accordingly, a lithium battery may exhibit enhanced performance and lifespan characteristics.

In addition, the unsaturated sulfone compound of Formula 3 may form, on the surface of the negative electrode, a stable SEI film capable of capturing Ni cations eluted from the positive electrode to thereby protect the negative electrode. Thus, when the electrolyte including the alkyl sulfonate compound and the unsaturated sulfone compound is applied, a lithium battery may have a reduced initial resistance and an increase in resistance of the lithium battery when stored at a high temperature may be lowered, and accordingly, the lithium battery may exhibit enhanced lifespan characteristics.

According to one embodiment, the electrolyte may not include an aromatic hydrocarbon or an aromatic hydrocarbon compound substituted with a halogen.

The electrolyte includes a lithium salt. For the lithium salt, refer to the descriptions provided herein.

The electrolyte includes a non-aqueous solvent. For the non-aqueous solvent, refer to the descriptions provided herein.

According to one embodiment, as described above, the electrolyte includes a cyclic carbonate compound, a cyclic acid anhydride compound, or a mixture thereof, and the unsaturated sulfone compound may have a higher reduction potential than that of the cyclic carbonate compound or the cyclic acid anhydride compound.

According to an embodiment, there is provided a lithium battery including a positive electrode, a negative electrode, and an electrolyte between the positive electrode and the negative electrode,
wherein the electrolyte includes a lithium salt, a non-aqueous solvent, the alkyl sulfonate compound, and the unsaturated sulfone compound, and
the positive electrode includes a positive electrode active material represented by Formula 1B below:

   <Formula 1B> LiₚNi_{q}M_{1-q}O₂₋ᵣAᵣ

   wherein, in Formula 1B, 0.9≤p≤1.2, 0.7≤q≤0.98, and 0≤r<0.2 are satisfied;
   M is at least one element selected from the group consisting of Al, Mg, Mn, Co, Fe, Cr, V, Ti, Cu, B, Ca, Zn, Zr, Nb, Mo, Sr, Sb, W, and Bi; and
   A is an element having an oxidation number of -1, -2, or -3.

According to one embodiment, the amount of the electrolyte in the lithium battery may be in a range of about 1 g/Ah to about 3 g/Ah.

The positive electrode includes the positive electrode active material of Formula 1B.

For example, in Formula 1B, A may be one of a halogen, S, and N, but the present disclosure is not limited thereto.

In Formula 1B, q denotes the content of Ni in the positive electrode active material. According to one embodiment, in Formula 1B, q may satisfy the following condition: 0.7≤q≤0.98.

<Formula 1B> LiₚNi_{q}M_{1-q}O₂₋ᵣAᵣ

wherein, in Formula 1B, 0.9≤p≤1.2, 0.7≤q≤0.98, and 0≤r<0.2 are satisfied;
M is at least one element selected from the group consisting of I, Mg, Mn, Co, Fe, Cr, V, Ti, Cu, B, Ca, Zn, Zr, Nb, Mo, Sr, Sb, W, and Bi; and
A is an element having an oxidation number of -1, -2, or -3.

According to one embodiment, in Formula 1B, q may satisfy the following condition: 0.8≤q≤0.98.

For example, the positive electrode may include at least one selected from Li_{1.02}Ni_{0.80}Co_{0.15}Mn_{0.05}O₂, Li_{1.02}Ni_{0.85}Co_{0.1}Mn_{0.05}O₂, Li_{1.02}Ni_{0.88}Co_{0.08}Mn_{0.04}O₂, Li_{1.02}Ni_{0.88}Co_{0.10}Mn_{0.02}O₂, Li_{1.02}Ni_{0.91}Co_{0.06}Mn_{0.03}O₂, LiNi_{0.94}Co_{0.04}Mn_{0.02}O₂, Li_{1.02}Ni_{0.80}Co_{0.15}Al_{0.05}O₂, Li_{1.02}Ni_{0.85}Co_{0.1}Al_{0.05}O₂, Li_{1.02}Ni_{0.88}Co_{0.08}Al_{0.04}O₂, Li_{1.02}Ni_{0.88}Co_{0.10}Al_{0.02}O₂, Li_{1.02}Ni_{0.91}Co_{0.06}Al_{0.03}O₂, and LiNi_{0.94}Co_{0.04}Al_{0.02}O₂.

According to one embodiment, the positive electrode active material of Formula 1B may be represented by Formula 30 or 40:

<Formula 30> Li_{p'}Ni_{q'}Co_{n-q'-r'}Al_{r'}O₂

<Formula 40> Li_{p'}Ni_{q'}Co_{1-q'-r'}Mn_{r'}O₂

wherein, in Formulae 30 and 40, 0.9≤p'≤1.2, 0.88≤q'≤0.98, 0<r'<0.1, 0<1-q'-r'<0.2 are satisfied.

For example, the positive electrode may include at least one selected from Li_{1.02}Ni_{0.88}Co_{0.08}Mn_{0.04}O₂, Li_{1.02}Ni_{0.88}Co_{0.10}Mn_{0.02}O₂, Li_{1.02}Ni_{0.91}Co_{0.06}Mn_{0.03}O₂, LiNi_{0.94}Co_{0.04}Mn_{0.02}O₂, Li_{1.02}Ni_{0.88}Co_{0.08}Al_{0.04}O₂, Li_{1.02}Ni_{0.88}Co_{0.10}Al_{0.02}O₂, Li_{1.02}Ni_{0.91}Co_{0.06}Al_{0.03}O₂, and LiNi_{0.94}Co_{0.04}Al_{0.02}O₂.

As described above, when a lithium metal oxide having a high Ni content is used, a high-capacity battery may be manufactured, but batteries of the related art exhibit poor lifespan characteristics as the amount of Ni cations increases.

In addition, as described below, gas is generated in a lithium battery including a negative electrode active material including a metal alloyable with lithium or a carbon-based negative electrode active material due to catalytic action at a high temperature, and accordingly, the lithium battery exhibits deteriorated lifespan characteristics.

As described above, when the amount of FEC, VC, VEC, MA, SA, the P-containing compound, or the S-containing compound is within the above range, a passivation film, i.e., an SEI film, including a chemical reaction product of this material may be formed on a part or all of the surface of the negative electrode.

In this regard, the unsaturated sulfone compound may form a rigid SEI film capable of capturing Ni cations eluted from the positive electrode. In addition, since the alkyl sulfonate compound and the unsaturated sulfone compound are included in the electrolyte, Ni cations are stabilized, and thus gas generation when stored at a high temperature and an increase in resistance due to the degradation of an SEI film may be increased, and accordingly, a battery may exhibit enhanced safety, lifespan, and performance.

In addition, the positive electrode may further include, in addition to the above-listed positive electrode active materials, at least one selected from the group consisting of lithium cobalt oxide, lithium nickel cobalt manganese oxide, lithium nickel cobalt aluminum oxide, lithium iron phosphate, and lithium manganese oxide, but the present disclosure is not particularly limited thereto, and the positive electrode may further include any positive electrode active material that may be used in the art.

The negative electrode includes a negative electrode active material, and the negative electrode active material may include at least one selected from a silicon-based compound, a carbon-based compound, a composite of a silicon-based compound and a carbon-based compound, and a silicon oxide (SiOₓ₁ where 0<x1<2). In another embodiment, for example, the negative electrode may include a negative electrode active material including a metal alloyable with lithium, a silicon-based negative electrode active material, and/or a carbon-based negative electrode active material.

For example, the silicon-based compound may include silicon particles having an average diameter of 200 nm or less.

For example, the carbon-based compound may include graphite.

For example, the composite of a silicon-based compound and a carbon-based compound may be a composite having a structure in which silicon nanoparticles are arranged on the carbon-based compound, a composite in which silicon particles are included on a surface of and inside the carbon-based compound, or a composite in which silicon particles are coated with the carbon-based compound and thereby included in the carbon-based compound. The composite of a silicon-based compound and a carbon-based compound may be an active material obtained by dispersing silicon nanoparticles having an average diameter of about 200 nm or less on carbon-based compound particles, and then coating the resulting structure with carbon, an active material in which silicon particles are present on and inside graphite, or the like. Secondary particles of the composite of a silicon-based compound and a carbon-based compound may have an average diameter of about 5 µm to about 20 µm, and silicon nanoparticles may have an average diameter of 200 nm or less, 150 nm or less, 100 nm or less, 50 nm or less, 20 nm or less, or 10 nm or less. For example, the average diameter of the silicon nanoparticles may be in a range of about 100 nm to about 150 nm.

For example, the composite of a silicon-based compound and a carbon-based compound may have a capacity of about 300 mAh/g to about 700 mAh/g. For example, the capacity of the composite of a silicon-based compound and a carbon-based compound may be in a range of about 400 mAh/g to about 600 mAh/g.

The lithium battery may have a capacity retention rate of 80% or higher, for example, 85% or higher, after 100 cycles of charging and discharging at 25 °C. For example, when the negative electrode of the lithium battery includes a silicon compound or a silicon oxide, the capacity retention rate of the lithium battery after 100 cycles of charging and discharging at 25 °C may be 90% or higher.

The lithium battery may have a DCIR increase rate of 130% or less after 300 cycles of charging and discharge at 25 °C. For example, when the negative electrode of the lithium battery includes a silicon compound or a silicon oxide, the DCIR increase rate of the lithium battery after 200 cycles of charging and discharge at 25 °C may be 125% or less, for example, 120% or less.

The lithium battery may have a DCIR increase rate of 130% or less after being stored at 60 °C for 30 days. For example, the DCIR increase rate of the lithium battery after being stored at 60 °C for 10 days may be 110% or less.

The lithium battery may have a cell energy density per unit volume of 500 Wh/L or more. Since the lithium battery provides a high energy density, i.e., 500 Wh/L or higher, high output may be obtained.

The type of the lithium battery is not particularly limited, and the lithium battery includes a lithium-ion battery, a lithium-ion polymer battery, a lithium sulfur battery, and the like.

A lithium battery according to an embodiment may be manufactured using the following method.

First, a positive electrode is prepared.

For example, a positive electrode active material, a conductive material, a binder, and a solvent are mixed to prepare a positive electrode active material composition. The positive electrode active material composition may be directly coated onto a positive electrode current collector to thereby complete the manufacture of a positive electrode. In another embodiment, the positive electrode active material composition may be cast onto a separate support and a film separated from the support may be laminated on a metal current collector to thereby complete the manufacture of a positive electrode. The positive electrode is not limited to the above-described form, and may have a form other than the above-described form.

The positive electrode active material may be used in combination with general lithium-containing metal oxides, in addition to the positive electrode active material of Formula 1A or the positive electrode active material of Formula 1B. The lithium-containing metal oxides may be, for example, two or more composite oxides of lithium and a metal selected from Co, Mn, Ni, and a combination thereof.

For example, the positive electrode active material may further include a compound represented by any one of formulae: LiₐA_{1-b}B_{b}D₂ where 0.90 ≤ a ≤ 1.8 and 0 ≤ b ≤ 0.5; LiₐE_{1-b}B_{b}O_{2-c}D_{c} where 0.90 ≤ a ≤ 1.8, 0 ≤ b ≤ 0.5, and 0 ≤ c ≤ 0.05; LiE_{2-b}B_{b}O_{4-c}D_{c} where 0 ≤ b ≤ 0.5 and 0 ≤ c ≤ 0.05; LiₐNi_{1-b-c}Co_{b}B_{c}D_{α} where 0.90 ≤ a ≤ 1.8, 0 ≤ b ≤ 0.5, 0 ≤ c ≤ 0.05, and 0 < α ≤ 2; LiₐNi_{1-b-c}Co_{b}B_{c}O_{2-α}F_{α} where 0.90 ≤ a ≤ 1.8, 0 ≤ b ≤ 0.5, 0 ≤ c ≤ 0.05, and 0 < α < 2; LiₐNi_{1-b-c}Co_{b}B_{c}O_{2-α}F₂ where 0.90 ≤ a ≤ 1.8, 0 ≤ b ≤ 0.5, 0 ≤ c ≤ 0.05, and 0 < α < 2; LiₐNi_{1-b-c}Mn_{b}B_{c}D_{α} where 0.90 ≤ a ≤ 1.8, 0 ≤ b ≤ 0.5, 0 ≤ c ≤ 0.05, and 0 < α ≤ 2; LiₐNi_{1-b-c}MnₚB_{c}O_{2-α}F_{α} where 0.90 ≤ a ≤ 1.8, 0 ≤ b ≤ 0.5, 0 ≤ c ≤ 0.05, and 0 < α < 2; LiₐNi_{1-b-c}Mn_{b}B_{c}O_{2-α}F₂ where 0.90 ≤ a ≤ 1.8, 0 ≤ b ≤ 0.5, 0 ≤ c ≤ 0.05, and 0 < α < 2; LiₐNi_{b}E_{c}G_{d}O₂ where 0.90 ≤ a ≤ 1.8, 0 ≤ b ≤ 0.9, 0 ≤ c ≤ 0.5, and 0.001 ≤ d ≤ 0.1; LiₐNi_{b}Co_{c}Mn_{d}GeO₂ where 0.90 ≤ a ≤ 1.8, 0 ≤ b ≤ 0.9, 0 ≤ c ≤ 0.5, 0 ≤ d ≤0.5, and 0.001 ≤ e ≤ 0.1; LiₐNiG_{b}O₂ where 0.90 ≤ a ≤ 1.8 and 0.001 ≤ b ≤ 0.1; LiₐCoG_{b}O₂ where 0.90 ≤ a ≤ 1.8 and 0.001 ≤ b ≤ 0.1; LiₐMnG_{b}O₂ where 0.90 ≤ a ≤ 1.8 and 0.001 ≤ b ≤ 0.1; LiₐMn₂G_{b}O₄ where 0.90 ≤ a ≤ 1.8 and 0.001 ≤ b ≤ 0.1; QO₂; QS₂; LiQS₂; V₂O₅; LiV₂O₅; LilO₂; LiNiVO₄; Li_{(3-f)}J₂(PO₄)₃ where 0 ≤ f ≤ 2; Li_{(3-f)}Fe₂(PO₄)₃ where 0 ≤ f ≤ 2; and LiFePO₄.

In the above formulae, A is Ni, Co, Mn, or a combination thereof; B is Al, Ni, Co, Mn, Cr, Fe, Mg, Sr, V, a rare earth element, or a combination thereof; D is oxygen (O), fluorine (F), sulfur (S), phosphorus (P), or a combination thereof; E is Co, Mn, or a combination thereof; F is F, S, P, or a combination thereof; G is Al, Cr, Mn, Fe, Mg, lanthanum (La), cerium (Ce), strontium (Sr), V, or a combination thereof; Q is titanium (Ti), molybdenum (Mo), Mn, or a combination thereof; I is Cr, V, Fe, scandium (Sc), yttrium (Y), or a combination thereof; and J is V, Cr, Mn, Co, Ni, copper (Cu), or a combination thereof.

For example, the positive electrode active material may be LiCoO₂, LiMnₓO₂ₓ where x=1 or 2, LiNi₁₋ₓMnₓO₂ₓ where 0<x<1, LiNi_{1-x-y}COₓMn_{y}O₂ where 0≤x≤0.5, 0≤y≤0.5, and 1-x-y>0.5, LiFePO₄, or the like.

The above-listed compounds having a coating layer on their surfaces may also be used, or mixtures of the above-listed compounds and the compounds having a coating layer may be used. The coating layer may include a coating element compound such as an oxide, a hydroxide, an oxyhydroxide, an oxycarbonate, or a hydroxycarbonate of a coating element. The compounds constituting the coating layer may be amorphous or crystalline. The coating element included in the coating layer may be Mg, Al, Co, potassium (K), sodium (Na), calcium (Ca), silicon (Si), Ti, V, tin (Sn), germanium (Ge), gallium (Ga), boron (B), arsenic (As), zirconium (Zr), or a mixture thereof. The coating layer may be formed using any coating method that does not adversely affect the physical properties of the positive electrode active material by using these coating elements in the above compounds (e.g., spray coating, dipping, or the like). The coating methods may be well understood by one of ordinary skill in the art, and thus a detailed description thereof is omitted.

The positive electrode active material composition may further include a conductive material, a filler, or the like.

The conductive material is generally added in an amount of about 1 wt% to about 30 wt% with respect to a total weight of a mixture including a positive electrode active material. The conductive material is not particularly limited as long as it does not cause any chemical change in the fabricated battery and has conductivity, and non-limiting examples thereof include: graphite such as natural graphite or artificial graphite; carbon black, acetylene black, Ketjen black, channel black, furnace black, lamp black, and thermal black; conductive fibers such as carbon fiber or metallic fiber; carbon fluoride; metallic powders such as aluminum powder and nickel powder; conductive whiskers such as zinc oxide and potassium titanate; conductive metal oxides such as titanium oxide; and polyphenylene derivatives.

The binder is a component assisting in binding between an active material and the conductive material and in binding between an active material and a current collector, and is generally added in an amount of about 1 wt% to about 30 wt% with respect to the total weight of the positive electrode active material composition. Non-limiting examples of the binder include polyvinylidenefluoride (PVdF), , polyvinylidenechloride, polybenzimidazole, polyimide, polyvinylacetate, polyacrylonitrile, polyvinylalcohol, carboxymethylcellulose (CMC), starch, hydroxypropylcellulose, regenerated cellulose, polyvinylpyrrolidone, polyethylene, polypropylene, polystyrene, polymethylmethacrylate, polyaniline, acrylonitrilebutadienestyrene, phenol resin, epoxy resin, polyethylenetelethphalate, polytetrafluoroethylene, polyphenylsulfide, polyamideimide, polyetherimide, polyethylenesulfone, polyamide, polyacetal, polyphenyleneoxide, polybutylenetelephthalate, ethylene-propylene-diene terpolymer (EPDM), sulfonated EPDM, styrene butadiene rubber (SBR), fluorine rubber, and various copolymers. The filler is a component that suppresses the expansion of a positive electrode and optionally used, and is not particularly limited as long as it does not cause a chemical change in the fabricated battery and is a fibrous material. Non-limiting examples of the filler include olefin-based polymers such as polyethylene and polypropylene; and fibrous materials such as glass fiber and carbon fiber.

As the solvent, N-methylpyrrolidone, acetone, water, or the like may be used, but the present disclosure is not limited thereto, and any solvent that may be used in the art may be used. The amount of the solvent may be in a range of, for example, about 10 parts by weight to about 100 parts by weight with respect to 100 parts by weight of the positive electrode active material. When the amount of the solvent is within the above range, the formation of an active material layer is facilitated.

The amounts of the positive electrode active material, the conductive material, the filler, the binder, and the solvent are the same as those used in general lithium batteries. At least one of the conductive material, the filler, the binder, and the solvent may be omitted depending on the use and configuration of a lithium battery.

For example, N-methylpyrrolidone (NMP) may be used as a solvent, PVdF or a PVdF copolymer may be used as a binder, and carbon black or acetylene black may be used as a conductive material. For example, 94 wt% of a positive electrode active material, 3 wt% of a binder, and 3 wt% of a conductive material may be mixed in a powder form, NMP may be added to the resulting mixture to prepare a slurry in which the solid content is 70 wt%, and the slurry may be coated, dried, and pressed, thereby completing the fabrication of a positive electrode.

The positive electrode current collector is generally fabricated to have a thickness of about 3 µm to about 50 µm. The positive electrode current collector is not particularly limited as long as it causes no chemical change in the fabricated battery and has high conductivity. Non-limiting examples of the positive electrode current collector include stainless steel; aluminum; nickel; titanium; sintered carbon; and copper or stainless steel that is surface-treated with carbon, nickel, titanium, or silver. In addition, the positive electrode current collector may be processed to have fine irregularities on surfaces thereof so as to enhance the adhesion of the positive electrode current collector to the positive electrode active material, and may be used in any of various forms including films, sheets, foils, nets, porous structures, foams, and non-woven fabrics.

For example, the positive electrode is manufactured by coating a positive electrode active material onto a positive electrode current collector and drying and pressing the resulting structure, and a positive electrode active material composition is prepared by mixing the above-described positive electrode active material and optionally, a binder and a solvent. The positive electrode active material composition is directly coated onto a metal current collector and dried, thereby completing the fabrication of a positive electrode plate. In another embodiment, the positive electrode active material composition may be cast on a separate support, and a film separated from the support may be laminated on a metal current collector, thereby completing the fabrication of a positive electrode plate.

For example, the prepared positive electrode active material composition may have a loading level of 30 mg/cm² or higher, for example, 35 mg/cm² or higher, for example, 40 mg/cm² or higher. In addition, the positive electrode active material composition may have an electrode density of 3 g/cc or more, for example, 3.5 g/cc or more.

In one embodiment, to achieve a high cell energy density, the prepared positive electrode active material composition may have a loading level of about 35 mg/cm² to about 50 mg/cm² and an electrode density of about 3.5 g/cc to about 4.2 g/cc.

In another embodiment, the positive electrode active material composition may be coated on opposite surfaces of the positive electrode plate to a loading level of 37 mg/cm² and an electrode density of 3.6 g/cc.

When the loading level and the electrode density of the positive electrode active material composition are within the above ranges, a battery including the positive electrode active material may exhibit a high cell energy density, i.e., 500 wh/L or higher. For example, the battery may exhibit a cell energy density of 500 wh/L to 900 wh/L.

Next, a negative electrode is prepared.

For example, a negative electrode active material, a conductive material, a binder, and a solvent are mixed to prepare a negative electrode active material composition.

The negative electrode is manufactured by coating a negative electrode active material onto a negative electrode current collector and drying and pressing the resulting structure, and a negative electrode active material composition is prepared by mixing the above-described positive electrode active material and optionally, a binder and a solvent.

For example, the negative electrode active material composition is directly coated onto a negative electrode current collector and dried, thereby completing the fabrication of a negative electrode. In another embodiment, the negative electrode active material composition may be cast on a separate support, and a film separated from the support may be laminated on a metal current collector, thereby completing the fabrication of a negative electrode plate.

The negative electrode active material may be, for example, a silicon-based compound, a silicon oxide (SiOₓ wherein 0<x<2), or a composite of a silicon-based compound and a carbon-based material. In this regard, silicon particles has a size (e.g., an average diameter) of less than 200 nm, for example, about 10 nm to about 150 nm. The term "size" as used herein refers to an average diameter of silicon particles when the silicon particles are spherical, and refers to an average length of major axes when the silicon particles are non-spherical.

When the size of the silicon particles is within the above range, excellent lifespan characteristics may be provided, and thus when an electrolyte according to an embodiment is used, a lithium battery may exhibit further enhanced lifespan.

The carbon-based material may be crystalline carbon, amorphous carbon, or a mixture thereof. The crystalline carbon may be graphite, such as natural graphite or artificial graphite that is in amorphous, plate, flake, spherical or fibrous form. The amorphous carbon may be soft carbon (carbon sintered at low temperatures), hard carbon, mesophase pitch carbides, sintered corks, or the like.

The composite of a silicon-based compound and a carbon-based material may be, for example, a composite having a structure in which silicon particles are arranged on graphite or a composite in which Si particles are included on a surface of and inside graphite. The composite may be, for example, an active material obtained by dispersing Si particles having an average diameter of about 200 nm or less, for example, about 100 nm to about 200 nm, for example, 150 nm, on graphite particles and performing carbon coating thereon, or an active material in which Si particles are present on and inside graphite. These composites are available under the trade name SCN1 (Si particles on Graphite) or SCN2 (Si particles inside as well as on Graphite). SCN1 is an active material obtained by dispersing Si particles having an average diameter of about 150 nm on graphite particles and then performing carbon coating thereon. SCN2 is an active material in which Si particles having an average diameter of about 150 nm are present on and inside graphite.

The negative electrode active material may be used in combination with any negative electrode active material of a lithium battery that may be used in the art, in addition to the above-described negative electrode active material. Examples of the negative electrode active material include Si, Sn, Al, Ge, Pb, Bi, Sb, a Si-Y' alloy (where Y' is an alkali metal, an alkali earth metal, Groups 13 to 16 elements, a transition metal, a transition metal oxide, a rare earth element, or a combination thereof except for Si), and a Sn-Y' alloy (where Y' is an alkali metal, an alkali earth metal, Groups 13 to 16 elements, a transition metal, a rare earth element, or a combination thereof except for Sn). Y may be magnesium (Mg), calcium (Ca), strontium (Sr), barium (Ba), radium (Ra), scandium (Sc), yttrium (Y), titanium (Ti), zirconium (Zr), hafnium (Hf), rutherfordium (Rf), vanadium (V), niobium (Nb), tantalum (Ta), dubnium (Db), chromium (Cr), molybdenum (Mo), tungsten (W), seaborgium (Sg), technetium (Tc), rhenium (Re), bohrium (Bh), iron (Fe), lead (Pb), ruthenium (Ru), osmium (Os), hassium (Hs), rhodium (Rh), iridium (Ir), palladium (Pd), platinum (Pt), copper (Cu), silver (Ag), gold (Au), zinc (Zn), cadmium (Cd), boron (B), aluminum (Al), gallium (Ga), tin (Sn), indium (In), germanium (Ge), phosphorus (P), arsenic (As), antimony (Sb), bismuth (Bi), sulfur (S), selenium (Se), tellurium (Te), polonium (Po), or combinations thereof.

For example, the negative electrode active material may be lithium titanium oxide, vanadium oxide, lithium vanadium oxide, or the like.

The negative electrode active material composition may further include a conductive material, a filler, or the like.

Meanwhile, in the negative electrode active material composition, the binder, the solvent, the conductive material, and the filler may be the same as those used in the positive electrode active material composition.

However, in the negative electrode active material composition, water may be used as a solvent. For example, water may be used as a solvent, carboxymethylcellulose (CMC), styrene butadiene rubber (SBR), an acrylate-based polymer, or a methacrylate-based polymer may be used as a binder, and carbon black, acetylene black, or graphite may be used as a conductive material.

The amounts of the negative electrode active material, the conductive material, the binder, and the solvent are those levels commonly used in lithium batteries. At least one of the conductive material, the binder, and the solvent may be omitted depending on the use and configuration of a lithium battery.

For example, 94 wt% of a negative electrode active material, 3 wt% of a binder, and 3 wt% of a conductive material may be mixed in a powder form, water may be added to the resulting mixture such that the content of solids becomes 70 wt% to thereby prepare a slurry, and the slurry may be coated, dried, and pressed, thereby completing the fabrication of a negative electrode plate.

The negative electrode current collector is generally fabricated to have a thickness of about 3 µm to about 50 µm. The negative electrode current collector is not particularly limited as long as it causes no chemical change in the fabricated battery and has conductivity. Non-limiting examples of the negative electrode current collector include copper; stainless steel; aluminum; nickel; titanium; sintered carbon; copper or stainless steel that is surface-treated with carbon, nickel, titanium, or silver; and aluminum-cadmium alloys. In addition, as in the positive electrode current collector, the negative electrode current collector may be processed to have fine irregularities on surfaces thereof so as to enhance the adhesion of the negative electrode current collector to the negative electrode active material, and may be used in any of various forms including films, sheets, foils, nets, porous structures, foams, and non-woven fabrics.

The loading level of the prepared negative electrode active material composition is set in accordance with the loading level of the positive electrode active material composition.

For example, depending on capacity/g of the negative electrode active material composition, the loading level may be 12 mg/cm² or more, for example, 15 mg/cm² or more. In addition, the electrode density of the negative electrode active material composition may be 1.5 g/cc or more, for example, 1.6 g/cc or more.

In one embodiment, to achieve a high cell energy density, the prepared negative electrode active material composition may have a loading level of about 15 mg/cm² to about 25 mg/cm² and an electrode density of about 1.6 g/cc to about 2.3 g/cc.

When the loading level and the electrode density of the negative electrode active material are within the above ranges, a battery including this negative electrode active material may exhibit a high cell energy density, i.e., 600 wh/L or higher.

Next, a separator to be inserted between the positive electrode and the negative electrode is prepared.

The separator may be any separator commonly used in lithium batteries. A separator having low resistance to ion transfer in an electrolyte and having an excellent electrolyte-retaining ability may be used. For example, the separator may be composed of one selected from glass fiber, polyester, Teflon, polyethylene, polypropylene, polytetrafluoroethylene (PTFE), and a combination thereof, each of which may be a non-woven or woven fabric. For example, a windable separator made of polyethylene, polypropylene, or the like may be used in lithium-ion batteries, and a separator having an excellent electrolyte-impregnating ability may be used in lithium-ion polymer batteries. For example, the separator may be manufactured according to the following method.

A polymer resin, a filler, and a solvent are mixed to prepare a separator composition. The separator composition may be directly coated onto an electrode and dried to thereby form a separator. In another embodiment, the separator composition may be cast on a support and dried, and then a separator film separated from the support may be laminated on an electrode, thereby completing the formation of a separator.

The polymer resin used in the formation of a separator is not particularly limited, and all materials used in a binder of an electrode plate may be used. For example, the polymer resin may be a vinylidene fluoride/hexafluoropropylene copolymer, PVdF, polyacrylonitrile, polymethylmethacrylate, a mixture thereof, or the like.

Next, the above-described electrolyte is prepared.

According to one embodiment, the electrolyte may further include, in addition to the above-described electrolyte, a non-aqueous electrolytic solution, a solid electrolyte, or an inorganic solid electrolyte.

Non-limiting examples of the organic solid electrolyte include polyethylene derivatives, polyethylene oxide derivatives, polypropylene oxide derivatives, phosphoric acid ester polymers, polyester sulfide, polyvinyl alcohol, and polyvinylidene fluoride, and polymers having ionic dissociation groups.

Non-limiting examples of the inorganic solid electrolyte include Li₃N, Lil, Li₅NI₂, Li₃N-LiI-LiOH, LiSiO₄, Li₂SiS₃, Li₄SiO₄, Li₄SiO₄-LiI-LiOH, and Li₃PO₄-Li₂S-SiS₂.

As illustrated in FIG. 1, a lithium battery 1 includes a positive electrode 3, a negative electrode 2, and a separator 4. The positive electrode 3, the negative electrode 2, and the separator 4 are wound or folded, and then accommodated in a battery case 5. Subsequently, an electrolyte is injected into the battery case 5 and the battery case 5 is sealed by a cap assembly 6 to thereby complete the manufacture of the lithium battery 1. The battery case 5 may have a cylindrical, rectangular, or thin-film form, or the like. The lithium battery 1 may be a lithium secondary battery. For example, the lithium secondary battery may be a large-scale thin-film-type battery. The lithium secondary battery may be a lithium-ion battery.

A separator may be disposed between the positive electrode and the negative electrode to form a battery assembly. A plurality of battery assemblies may be stacked in a bi-cell structure and impregnated with an electrolyte, and the resultant structure may be put into a pouch and hermetically sealed to thereby complete the manufacture of a lithium-ion polymer battery.

In addition, the battery assemblies may be stacked to form a battery pack, and such a battery pack may be used in any device requiring high capacity and high power output. For example, the battery pack may be used in notebook computers, smartphones, electric vehicles, and the like.

A lithium secondary battery according to an embodiment may exhibit excellent characteristics due to a significantly reduced DCIR increase rate thereof, compared to a general lithium secondary battery employing a Ni-rich lithium nickel composite oxide as a positive electrode active material.

An operating voltage of the lithium secondary battery including the positive electrode, the negative electrode, and the electrolyte may have, for example, a lower limit of about 2.5 V to about 2.8 and an upper limit of about 4.1 V to about 4.4 V, and the lithium secondary battery has an excellent energy density, i.e., 500 wh/L or higher.

In addition, examples of the lithium secondary battery include, but are not limited to, power tools driven by an electric motor; electric vehicles (EVs), hybrid EVs (HEVs), and plug-in HEVs (PHEVs); electric two-wheel vehicles including E-bikes and E-scooters; electric golf carts; and power storage systems.

As used herein, the term "alkyl" refers to completely saturated, branched or unbranched (or a straight or linear) hydrocarbon.

Non-limiting examples of the "alkyl" include methyl, ethyl, n-propyl, isopropyl, n-butyl, isobutyl, sec-butyl, n-pentyl, isopentyl, neopentyl, n-hexyl, 3-methylhexyl, 2,2-dimethylpentyl, 2,3-dimethylpentyl, and n-heptyl.

At least one hydrogen atom of the "alkyl" may be substituted with a halogen atom, a C₁-C₂₀ alkyl group substituted with a halogen atom (e.g., CCF₃, CHCF₂, CH₂F, CCl₃, or the like), a C₁-C₂₀ alkoxy group, a C₂-C₂₀ alkoxyalkyl group, a hydroxyl group, a nitro group, a cyano group, an amino group, an amidino group, hydrazine, hydrazone, a carboxyl group or a salt thereof, a sulfonyl group, a sulfamoyl group, sulfonic acid group or a salt thereof, phosphoric acid or a salt thereof, a C₁-C₂₀ alkyl group, a C₂-C₂₀ alkenyl group, a C₂-C₂₀ alkynyl group, a C₁-C₂₀ heteroalkyl group, a C₆-C₂₀ aryl group, a C₆-C₂₀ arylalkyl group, a C₆-C₂₀ heteroaryl group, a C₇-C₂₀ heteroarylakyl group, a C₆-C₂₀ heteroaryloxy group, a C₆-C₂₀ heteroaryloxyalkyl group, or a C₆-C₂₀ heteroarylalkyl group.

The term "halogen" as used herein includes fluorine, bromine, chloride, iodine, and the like.

The term "alkoxy" refers to "alkyl-O-", wherein the alkyl group is the same as described above. The alkoxy group may be, for example, methoxy group, an ethoxy group, a 2-propoxy group, a butoxy group, a t-butoxy group, a pentyloxy group, a hexyloxy group, or the like. At least one hydrogen atom of the alkoxy group may be substituted with the same substituent as in the above-described alkyl group.

The term "alkenyl" refers to branched or non-branched hydrocarbon having at least one carbon-carbon double bond. Non-limiting examples of the alkenyl group include vinyl, allyl, butenyl, propenyl, and isobutenyl, and at least one hydrogen atom of the alkenyl group may be substituted with the same substituent as in the above-described alkyl group.

The term "alkynyl" refers to branched or non-branched hydrocarbon having at least one carbon-carbon triple bond. Non-limiting examples of the "alkynyl" include ethynyl, butynyl, isobutynyl, and isopropynyl.

At least one hydrogen atom of the "alkynyl" may be substituted with the same substituent as in the above-described alkyl group.

The term "aryl" is intended to also include a group with an aromatic ring selectively fused to at least one carbon ring. Non-limiting examples of the "aryl" include phenyl, naphthyl, and tetrahydronaphthyl. In addition, at least one hydrogen atom of the "aryl" may be substituted with the same substituent as in the above-described alkyl group.

The term "heteroaryl" refers to a monocyclic or bicyclic organic group containing at least one heteroatom selected from N, O, P, or S, wherein the rest of the cyclic atoms is carbon. The heatoaryl group may include, for example, 1 to 5 heteroatoms and a five- to ten-membered ring. S or N may be present in various oxidized forms.

Examples of the heteroaryl group include thienyl, furyl, pyrrolyl, imidazolyl, pyrazolyl, thiazolyl, isothiazolyl, 1,2,3-oxadiazolyl, 1,2,4-oxadiazolyl, 1,2,5-oxadiazolyl, 1,3,4-oxadiaxolyl, 1,2,3-thiadiazolyl, 1,2,4-thiadiazolyl, 1,2,5-thiadiazolyl, 1,3,4-thiadiazolyl, isothiazol-3-yl, isothiazol-4-yl, isothiazol-5-yl, oxazol-2-yl, oxazol-4-yl, oxazol-5-yl, isoxazol-3-yl, isoxazol-4-yl, isoxazol-5-yl, 1,2,4-triazol-3-yl, 1,2,4-triazol-5-yl, 1,2,3-triazol-4-yl, 1,2,3-triazol-5-yl, tetrazolyl, pyrid-2-yl, pyrid-3-yl, 2-pyrazin-2-yl, pyrazin-4-yl, pyrazin-5-yl, 2-pyrimidin-2-yl, 4-pyrimidin-2-yl, and 5-pyrimidin-2-yl.

The term "heteroaryl" includes a heteroaromatic ring selectively fused to at least one aryl, cycloaliphatic or heterocyclic group.

Hereinafter, the present disclosure will be described in further detail with reference to the following examples and comparative examples. However, these examples are provided for illustrative purposes only and are not intended to limit the scope of the present disclosure.

### Examples 1 to 6 and Comparative Examples 1 to 14

Lithium batteries were manufactured in accordance with the configurations shown in Tables 2 to 6 below. Each configuration was manufactured in detail as follows.

### (Manufacture of Positive Electrode 1)

LiNi_{0.88}Co_{0.10}Mn_{0.02}O₂ as a positive electrode active material, carbon black as a conductive material, and PVdF as a binder were added into N-methylpyrrolidone (NMP) in a weight ratio of 97.7:1:1.1 and mixed, and then the resulting solution was coated onto opposite surfaces of Al foil having a thickness of 15 µm by dispersion to an area of 33 mg/cm² per surface, dried, and then pressed, thereby completing the manufacture of positive electrode 1 having an electrode density of 3.6 g/cc.

### (Manufacture of Negative Electrode 1)

Pitch-coated Si: Gr (weight ratio of 14.9: 81.1) as a negative electrode active material and an AG binder were added to NMP in a weight ratio of 96:4 and mixed, and the resulting solution was coated onto opposite surfaces of Cu foil having a thickness of 8 µm by dispersion to an area of 17.6 mg/cm² per surface, dried, and pressed, thereby completing the manufacture of negative electrode 1 having an electrode density of 1.65 g/cc.

### (Manufacture of Negative Electrode 2)

Pitch-free Si: Gr (weight ratio of 14.9: 81.1) as a negative electrode active material and an AG binder were added to NMP in a weight ratio of 96:4 and mixed, and the resulting solution was coated onto opposite surfaces of Cu foil having a thickness of 8 µm by dispersion to an area of 17.6 mg/cm² per surface, dried, and pressed, thereby completing the manufacture of negative electrode 2 having an electrode density of 1.65 g/cc.

### (Manufacture of Negative Electrode 3)

MC09:MC21:MC10 as a negative electrode active material (weight ratio of 20:70:10, available from Mitsubishi) and BM480B/BSH12 as a binder (weight ratio of 1.5:1.0, BM480B is manufactured by ZEON and BSH12 is manufactured by Dai-ichi Kogyo Seiyaku) were added to NMP in a weight ratio of 96:4 and mixed, and the resulting solution was coated onto opposite surfaces of Cu foil having a thickness of 8 µm by dispersion to an area of 17.6 mg/cm² per surface, dried, and pressed, thereby completing the manufacture of negative electrode 3 having an electrode density of 1.65 g/cc.

### (Preparation of Electrolyte)

As solvent 1, 1.15M LiPF₆ and FEC/EC/EMC/DMC (volume ratio: 3/10/47/40) was used. As solvent 2, 1 wt% of VC, 0.2 wt% of LiFSI, and 2 wt% of TMP were added to 1.3M LiPF₆ and FEC/EC/EMC/DMC (volume ratio: 7/10/13/70). As solvent 3, 1 wt% of VC, 0.2 wt% of LiFSI, and 2 wt% of TMP were added to 1.3M LiPF₆ and FEC/EC/EMC/DMC (volume ratio: 5/10/15/70). As additives added to solvents 1 to 3, additives shown in Tables 2 to 6 below were used to prepare an electrolyte. For the chemical structure of each additive, refer to Table 1 below.

**[Table 1]**

| Abbreviation | Chemical Name | Chemical structure |
|---|---|---|
| MMSN | Methyl methanesulfonate | 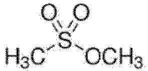 |
| EMS | Ethylmethane sulfonate | 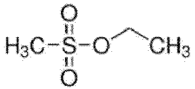 |
| PhpTs | Phenyl p-Toluenesulfonate | 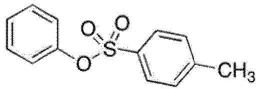 |
| PMSN | Phenyl methanesulfonate | 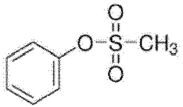 |
| EVS | Ethyl vinyl sulfone | 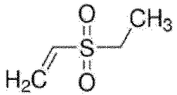 |
| PTSN | Propargyl p-toluenesulfonate | 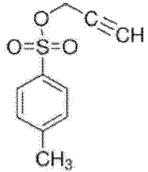 |
| AMS | Allyl methyl sulfone | 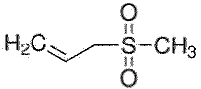 |
| TMP | Trimethylphosphate | 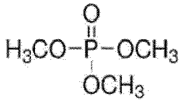 |
| EMSF | Ethyl methyl sulfone | 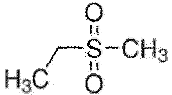 |
| DPhS | Diphenyl sulfone | 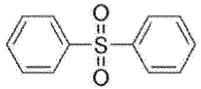 |

### (Assembly of Lithium Battery)

A separator made of polypropylene and having a thickness of 16 µm was disposed between the positive electrode and each negative electrode, and the electrolyte was injected thereinto, thereby completing the manufacture of a lithium battery.

### Evaluation Example 1: Evaluation of Gas Generation Amount and Direct Current Internal Resistance (DCIR) Characteristics

Each of the lithium batteries manufactured according to Examples 1 to 4 and Comparative Examples 1 to 9 was charged at a constant current of 0.2 C rate at 25 °C until the voltage reached 3.6 V (vs. Li), and discharged at a constant current of 0.2 rate until the voltage reached 2.8 V (vs. Li) (1^{st} cycle of formation operation). Subsequently, each lithium battery was charged at a constant current of 0.2 C rate until the voltage reached 4.25 V (vs. Li). Thereafter, each lithium battery was discharged at a constant current of 0.2 rate until the voltage reached 2.8 V (vs. Li) (2^{nd} cycle of formation operation). Third, each lithium battery was charged at a constant current of 0.5 C rate until the voltage reached 4.25 V (vs. Li) and then, while maintaining a constant voltage of 4.25 V, the charging process was cut off at a current of 0.05 C rate. Subsequently, each lithium battery was discharged at a constant current of 0.2 rate until the voltage reached 2.8 V (vs. Li). For the 4^{th} cycle, the 3^{rd} formation operation was repeated (0.5 C charging/0.2 C discharging). Lastly, each lithium battery was charged at a constant current of 0.2 C rate until the voltage reached 4.25 V and, while maintaining a constant voltage of 4.25 V, the charging process was cut off at a current of 0.05 C rate.

The expression "1C charging" means that battery capacity (mAh) is reached by charging for 1 hour. Likewise, the expression "1C discharging" means that the battery capacity (mAh) is completely consumed by discharging for 1 hour.

The lithium batteries having undergone the formation operations were maintained at 60 °C for 10 days to evaluate gas generation amounts and DCIR characteristics, and the results thereof are shown in Tables 2 and 3.

**[Table 2]**

| | Electrolyte | | Positive electrode | Negative electrode | Gas generation amount (@60 °C 10D) [Relative value (%)] | 0D DCIR (mΩ) | 10D DCIR (mΩ) | ΔDCIR (%) |
|---|---|---|---|---|---|---|---|---|
| | Solvent | Additive | | | | | | |
| Example 1 | Solvent 1 | 0.5wt% MMSN | Positive electrode 1 | Negative electrode 1 | 0.67 | 131 | 127 | 97 |
| Example 2 | Solvent 1 | 0.5wt% EMS | Positive electrode 1 | Negative electrode 1 | 0.75 | 133 | 129 | 97 |
| Comparative Example 1 | Solvent 1 | - | Positive electrode 1 | Negative electrode 1 | 0.78 | 130 | 130 | 100 |
| Comparative Example 2 | Solvent 1 | 0.5 wt% PhpTs | Positive electrode 1 | Negative electrode 1 | 0.72 | 137 | 140 | 108 |
| Comparative Example 3 | Solvent 1 | 0.5 wt% PMSN | Positive electrode 1 | Negative electrode 1 | 0.78 | 130 | 132 | 101 |
| Example 3 | Solvent 1 | 1wt% MMSN | Positive electrode 1 | Negative electrode 2 | 0.77 | 124 | 132 | 107 |
| Comparative Example 4 | Solvent 1 | 0.3 wt% PTSN + 0.3wt% MMSN | Positive electrode 1 | Negative electrode 2 | 0.77 | 122 | 133 | 109 |
| Comparative Example 5 | Solvent 1 | 1wt% sulfolane | Positive electrode 1 | Negative electrode 2 | 0.82 | 126 | 138 | 109 |

**[Table 3]**

| | Electrolyte | | Positive electrode | Negative electrode | Gas generation amount (@60 °C 10D) [Relative value (%)] | 0D DCIR (mΩ) | 10D DCIR (mΩ) | ΔDCIR (%) |
|---|---|---|---|---|---|---|---|---|
| | Solvent | Additive | | | | | | |
| Example 4 | Solvent 2 | 0.3 wt% EVS + 0.3wt% MMSN | Positive electrode 1 | Negative electrode 2 | 0.64 | 142 | 171 | 121 |
| Comparative Example 6 | Solvent 2 | - | Positive electrode 1 | Negative electrode 2 | 0.90 | 148 | 189 | 127 |
| Comparative Example 7 | Solvent 2 | 0.6 wt% EVS | Positive electrode 1 | Negative electrode 2 | 0.70 | 148 | 192 | 130 |
| Comparative Example 8 | Solvent 2 | 0.3 wt% EVS + 0.3wt% AMS | Positive electrode 1 | Negative electrode 2 | 0.79 | 150 | 198 | 132 |
| Comparative Example 9 | Solvent 2 | 0.3 wt% PTSN+ 0.3wt% MMSN | Positive electrode 1 | Negative electrode 2 | 0.73 | 148 | 192 | 129 |

Referring to Table 2, it was confirmed that the lithium batteries of Examples 1 and 2 exhibited gas generation amounts that were lower than or equivalent to those of the lithium batteries of Comparative Examples 1 to 3 including different additives under the same conditions, and exhibited significantly reduced DCIR increase rates, which indicates excellent stability. In particular, it was confirmed that the lithium secondary batteries of Examples 1 and 2 exhibited substantially lowered DCIRs when stored at a high temperature.

In addition, referring to Table 2, it was confirmed that the lithium battery of Example 3 exhibited significantly reduced gas generation and DCIR increase rate, which indicates excellent stability, as compared to the lithium batteries of Comparative Examples 4 and 5 including different additives under the same conditions.

In addition, referring to Table 3, it was confirmed that the lithium battery of Example 4 exhibited significantly reduced gas generation and DCIR increase rate, which indicates excellent stability, as compared to the lithium batteries of Comparative Examples 6 to 9 including different additives under the same conditions.

### Evaluation Example 2: Evaluation of Gas Generation Amount and DCIR Characteristics

Each of the lithium batteries of Examples 5 and 6 and Comparative Examples 10 and 11 was subjected to a formation operation in the same manner as in Evaluation Example 1, followed by 100 cycles of charging and discharging at 45 °C under conditions of a charging/discharging current of 1C/1C, an operating voltage of 2.8 V to 4.25 V, and cut-off at CC-CV 1/10C, and then the capacity retention rate and DCIR of each lithium battery were measured. The results thereof are shown in Table 4 below.

**[Table 4]**

| | Electrolyte | | Positive electrode | Negative electrode | Capacity retention rate (%, @45 °C, 100CY) | 0D DCIR (mΩ) | 10D DCIR (mQ) | ΔDCIR (%) |
|---|---|---|---|---|---|---|---|---|
| | Solvent | Additive | | | | | | |
| Example 5 | Solvent 3 | 0.3 wt% EVS + 0.3wt% MMSN | Positive electrode 1 | Negative electrode 2 | 91.6 | 144 | 150 | 104 |
| Comparative Example10 | Solvent 3 | 0.3 wt% PTSN + 0.3wt% MMSN | Positive electrode 1 | Negative electrode 2 | 91.6 | 149 | 161 | 108 |
| Example 6 | Solvent 3 | 0.3 wt% EVS + 0.3wt% MMSN | Positive electrode 1 | Negative electrode 2 | 74.6 | 144 | 182 | 126 |
| Comparative Example 11 | Solvent 3 | 0.3 wt% PTSN + 0.3wt% MMSN | Positive electrode 1 | Negative electrode 2 | 74.0 | 145 | 195 | 134 |

Referring to Table 4, the lithium batteries of Examples 5 and 6 exhibited substantially the same or a longer lifespan and significantly reduced DCIR increase rates compared to the lithium batteries of Comparative Examples 10 and 11 including different additives under the same conditions, from which it was confirmed that the lithium batteries of Examples 5 and 6 had excellent stability.

### Evaluation Example 3: Evaluation of Gas Generation Amount and DCIR Characteristics

Each of the lithium batteries of Comparative Examples 12 to 14 was charged and discharged in the same manner as in Evaluation Example 1, except that the operating voltage was adjusted to 2.8 V to 4.3 V, and then gas generation amount and DCIR of each lithium battery were measured, and the results thereof are shown in Table 5 below.

**[Table 5]**

| | Electrolyte | | Positive electrode | Negative electrode | Gas generation amount (@60 °C, 10D) | 0D DCIR (mΩ) | 10D DCIR (mΩ) | ΔDCIR (%) |
|---|---|---|---|---|---|---|---|---|
| | Solvent | Additive | | | | | | |
| Comparative Example 12 | Solvent 1 | 1.5wt% VC + 1wt% TMP | Positive electrode 1 | Negative electrode 3 | 0.62 | 191 | 219 | 114 |
| Comparative Example 13 | Solvent 1 | 1.5wt% VC+ 1wt% EMSF | Positive electrode 1 | Negative electrode 3 | 0.66 | 186 | 228 | 122 |
| Comparative Example 14 | Solvent 1 | 1.5wt% VC + 1wt% DPhS | Positive electrode 1 | Negative electrode 3 | 0.59 | 191 | 224 | 117 |

Referring to Table 5, it was confirmed that, when a sulfone compound was used instead of the unsaturated sulfone compound, the gas generation amount and/or the DCIR increase rate were further increased compared to the case of using TMP, which is an additive generally used in lithium batteries.

As is apparent from the foregoing description, use of an electrolyte including the alkyl sulfonate compound results in suppression of a side reaction of a lithium battery, and the lithium battery exhibits reduced gas generation and enhanced lifespan characteristics. In addition, an electrolyte including the alkyl sulfonate compound and the unsaturated sulfone compound is used, and thus a side reaction of a lithium battery is suppressed and the lithium battery exhibits reduced gas generation and enhanced lifespan characteristics.

It should be understood that embodiments described herein should be considered in a descriptive sense only and not for purposes of limitation. Descriptions of features or aspects within each embodiment should typically be considered as available for other similar features or aspects in other embodiments.

## Claims

1. A lithium battery comprising: a positive electrode; a negative electrode; and an electrolyte between the positive electrode and the negative electrode,
wherein the positive electrode comprises a positive electrode active material represented by Formula 1A below, and
the electrolyte comprises a lithium salt, a non-aqueous solvent, and an alkyl sulfonate compound represented by Formula 2 below:
<Formula 1A> LiₓNi_{y}M_{1-y}O_{2-z}A_{z}
wherein, in Formula 1A, 0.9≤x≤1.2, 0.88≤y≤0.98, and 0≤z<0.2 are satisfied,
M is at least one element selected from the group consisting of aluminum (Al), magnesium (Mg), manganese (Mn), cobalt (Co), iron (Fe), chromium (Cr), vanadium (V), titanium (Ti), copper (Cu), boron (B), calcium (Ca), zinc (Zn), zirconium (Zr), niobium (Nb), molybdenum (Mo), strontium (Sr), antimony (Sb), tungsten (W), and bismuth (Bi); and
A is an element having an oxidation number of -1 or -2; 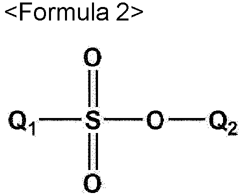
wherein, in Formula 2, Q₁ and Q₂ are each independently a substituted or unsubstituted C₁-C₂₀ alkyl group,
wherein an amount of the alkyl sulfonate compound is in a range of 0.005 parts by weight to 5 parts by weight with respect to 100 parts by weight of the electrolyte, and
wherein substituted means that at least one hydrogen atom is substituted with a halogen atom, a C₁-C₂₀ alkyl group substituted with a halogen atom, a C₁-C₂₀ alkoxy group, a C₂-C₂₀ alkoxyalkyl group, a hydroxyl group, a nitro group, a cyano group, an amino group, an amidino group, hydrazine, hydrazone, a carboxyl group or a salt thereof, a sulfonyl group, a sulfamoyl group, sulfonic acid group or a salt thereof, phosphoric acid or a salt thereof, a C₁-C₂₀ alkyl group, a C₂-C₂₀ alkenyl group, a C₂-C₂₀ alkynyl group, a C₁-C₂₀ heteroalkyl group, a C₆-C₂₀ aryl group, a C₆-C₂₀ arylalkyl group, a C₆-C₂₀ heteroaryl group, a C₇-C₂₀ heteroarylakyl group, a C₆-C₂₀ heteroaryloxy group, a C₆-C₂₀ heteroaryloxyalkyl group, or a C₆-C₂₀ heteroarylalkyl group.

2. The lithium battery of claim 1, wherein Q₁ and Q₂ are each independently selected from: a methyl group, an ethyl group, a propyl group, an isopropyl group, a butyl group, a sec-butyl group, a tert-butyl group, and an isobutyl group, and
a methyl group, an ethyl group, a propyl group, an isopropyl group, a butyl group, a sec-butyl group, a tert-butyl group, or an isobutyl group, each substituted with at least one selected from deuterium, -F, -Cl ,-Br, -I, a cyano group, a nitro group, a hydroxyl group, a methyl group, an ethyl group, and a propyl group.

3. The lithium battery of claims 1 or 2, wherein the alkyl sulfonate compound is selected from compounds 1 and 2 below: 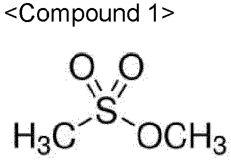 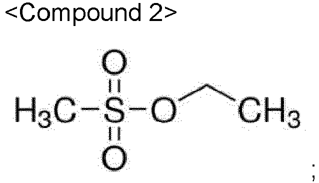 and/or
wherein the lithium salt comprises at least one selected from LiPF₆, LiBF₄, LiCF₃SO₃, Li(CF₃SO₂)₂N, LiC₂F₅SO₃, Li(FSO₂)₂N, LiC₄F₉SO₃, LiN(SO₂CF₂CF₃)₂, and compounds represented by Formulae 22 to 25 below: 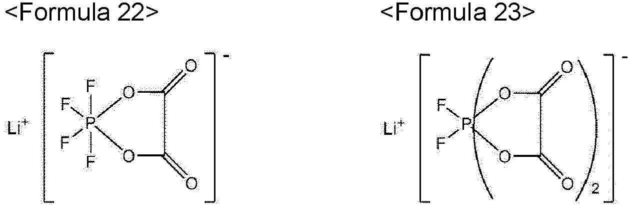 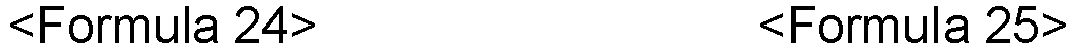 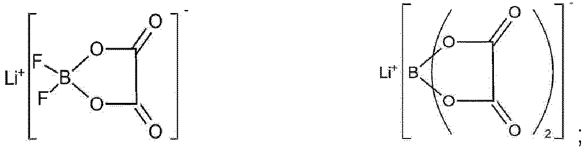 and/or
wherein the non-aqueous solvent is selected from dimethyl carbonate (DMC), diethyl carbonate (DEC), ethyl methyl carbonate (EMC), dipropyl carbonate (DPC), methyl propyl carbonate (MPC), ethyl propyl carbonate (EPC), methyl ethyl carbonate (MEC), ethylene carbonate (EC), propylene carbonate (PC), butylene carbonate (BC), methyl propionate (MP), ethyl propionate, propyl propionate (PP), tetraethylene glycol dimethyl ether (TEGDME), and mixtures thereof.

4. The lithium battery of any of claims 1-3, wherein the electrolyte further comprises one selected from fluoro-ethylene carbonate (FEC), vinylene carbonate (VC), vinyl ethylene carbonate (VEC), maleic anhydride, succinic anhydride, a phosphorus (P)-containing compound, a sulfur (S)-containing compound, and mixtures thereof,
wherein the P-containing compound comprises at least one selected from a phosphine compound, a phosphate compound, and a phosphite compound, and
the S-containing compound comprises at least one selected from a sulfone compound, a sulfonate compound, a sultone compound, and a disulfonate compound.

5. The lithium battery of any of claims 1-4, wherein the positive electrode active material is represented by Formula 10 or 20:
<Formula 10> Li_{x'}Ni_{y'}Co_{1-y'-z'}Al_{z'}O₂
<Formula 20> Li_{x'}Ni_{y'}Co_{1-y'-z'}Mn_{z'}O₂
wherein, in Formulae 10 and 20, 0.9≤x'≤1.2, 0.88≤y'≤0.98, 0<z'<0.1, and 0<1-y'-z'<0.2 are satisfied; and/or
wherein the positive electrode comprises at least one selected from Li_{1.02}Ni_{0.88}Co_{0.08}Mn_{0.04}O₂, Li_{1.02}Ni_{0.88}Co_{0.10}Mn_{0.02}O₂, Li_{1.02}Ni_{0.91}Co_{0.06}Mn_{0.03}O₂, LiNi_{0.94}Co_{0.04}Mn_{0.02}O₂, Li_{1.02}Ni_{0.88}Co_{0.08}Al_{0.04}O₂, Li_{1.02}Ni_{0.88}Co_{0.10}Al_{0.02}O₂, Li_{1.02}Ni_{0.91}Co_{0.06}Al_{0.03}O₂, and LiNi_{0.94}Co_{0.04}Al_{0.02}O₂; and/or
wherein the negative electrode comprises a negative electrode active material, the negative electrode active material comprising at least one selected from a silicon-based compound, a carbon-based compound, a composite of a silicon-based compound and a carbon-based compound, and a silicon oxide (SiOₓ₁ where 0<x1<2).

6. The lithium battery of any of claims 1-5, wherein the electrolyte further includes an unsaturated sulfone compound represented by Formula 3 below: 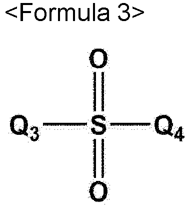
wherein, in Formula 3, Q₃ and Q₄ are each independently selected from a group represented by -(L₁)-(R₁), a vinyl group, an allyl group, and a substituted or unsubstituted C₁-C₂₀ alkyl group,
at least one of Q₃ and Q₄ is a group represented by -(L₁)-(R₁), a vinyl group, or an allyl group,
L₁ is selected from a substituted or unsubstituted C₂-C₂₀ alkenylene group and a substituted or unsubstituted C₂-C₂₀ alkynylene group, and
R₁ is selected from hydrogen and a substituted or unsubstituted C₂-C₂₀ alkyl group,
wherein substituted means that at least one hydrogen atom is substituted with a halogen atom, a C₁-C₂₀ alkyl group substituted with a halogen atom, a C₁-C₂₀ alkoxy group, a C₂-C₂₀ alkoxyalkyl group, a hydroxyl group, a nitro group, a cyano group, an amino group, an amidino group, hydrazine, hydrazone, a carboxyl group or a salt thereof, a sulfonyl group, a sulfamoyl group, sulfonic acid group or a salt thereof, phosphoric acid or a salt thereof, a C₁-C₂₀ alkyl group, a C₂-C₂₀ alkenyl group, a C₂-C₂₀ alkynyl group, a C₁-C₂₀ heteroalkyl group, a C₆-C₂₀ aryl group, a C₆-C₂₀ arylalkyl group, a C₆-C₂₀ heteroaryl group, a C₇-C₂₀ heteroarylakyl group, a C₆-C₂₀ heteroaryloxy group, a C₆-C₂₀ heteroaryloxyalkyl group, or a C₆-C₂₀ heteroarylalkyl group.

7. The lithium battery of claim 6, wherein an amount of the alkyl sulfonate compound is in a range of 0.001 parts by weight to 3 parts by weight with respect to 100 parts by weight of the electrolyte; and/or
wherein an amount of the unsaturated sulfone compound is in a range of 0.001 parts by weight to 3 parts by weight with respect to 100 parts by weight of the electrolyte.

8. The lithium battery of claims 6 or 7, wherein Q₁ and Q₂ are each independently selected from: a methyl group, an ethyl group, a propyl group, an isopropyl group, a butyl group, a sec-butyl group, a tert-butyl group, and an isobutyl group; and
a methyl group, an ethyl group, a propyl group, an isopropyl group, a butyl group, a sec-butyl group, a tert-butyl group, or an isobutyl group, each substituted with at least one selected from deuterium, -F, -Cl ,-Br, -I, a cyano group, a nitro group, a hydroxyl group, a methyl group, an ethyl group, and a propyl group, and
Q₃ and Q₄ are each independently selected from: a vinyl group, an allyl group, a methyl group, an ethyl group, a propyl group, an isopropyl group, a butyl group, a sec-butyl group, a tert-butyl group, and an isobutyl group; and
a methyl group, an ethyl group, a propyl group, an isopropyl group, a butyl group, a sec-butyl group, a tert-butyl group, or an isobutyl group, each substituted with at least one selected from deuterium, -F, -Cl ,-Br, -I, a cyano group, a nitro group, a hydroxyl group, a methyl group, an ethyl group, and a propyl group, and
at least one of Q₃ and Q₄ is a vinyl group or an allyl group.

9. The lithium battery of any of claims 6-8, wherein the alkyl sulfonate compound is selected from compounds 1 and 2 below: 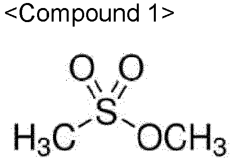 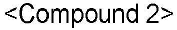 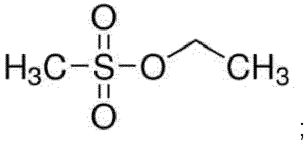 and/or
wherein the unsaturated sulfone compound is compound 3 below:

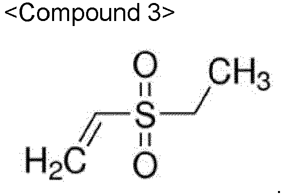

10. The lithium battery of any of claims 6-9, wherein the electrolyte does not comprise an aromatic hydrocarbon compound or an aromatic hydrocarbon compound substituted with a halogen.

11. The lithium battery of any of claims 6-10, wherein a concentration of the lithium salt in the electrolyte is in a range of 1.0 M to 1.5 M.

12. The lithium battery of any of claims 6-11, wherein the electrolyte further comprises one selected from fluoro-ethylene carbonate (FEC), vinylene carbonate (VC), vinyl ethylene carbonate (VEC), maleic anhydride, succinic anhydride, a phosphorus (P)-containing compound, a sulfur (S)-containing compound, and mixtures thereof,
wherein the P-containing compound comprises at least one selected from a phosphine compound, a phosphate compound, and a phosphite compound, and
the S-containing compound comprises at least one selected from a sulfone compound, a sulfonate compound, a sultone compound, and a disulfonate compound;
and wherein an amount of each of the FEC, VC, VEC, maleic anhydride, and succinic anhydride is in a range of 0.1 parts by weight to 2 parts by weight with respect to 100 parts by weight of the electrolyte.

## Patentansprüche

1. Lithiumbatterie, umfassend: eine positive Elektrode; eine negative Elektrode; und einen Elektrolyt zwischen der positiven Elektrode und der negativen Elektrode,
wobei die positive Elektrode ein aktives positives Elektrodenmaterial umfasst, dargestellt durch Formel 1A unten, und
der Elektrolyt ein Lithiumsalz, ein nicht-wässriges Lösungsmittel und eine Alkylsulfonatverbindung umfasst, dargestellt durch Formel 2 unten:
<Formel 1a> LiₓNi_{y}M_{1-y}O_{2-z}A_{z}
wobei in Formel 1A 0,9≤x≤1,2, 0,88≤y≤0,98 und 0≤z≤0,2 erfüllt sind,
M mindestens ein Element ist, ausgewählt aus der Gruppe, bestehend aus Aluminium (Al), Magnesium (Mg), Mangan (Mn), Kobalt (Co), Eisen (Fe), Chrom (Cr), Vanadium (V), Titan (Ti), Kupfer (Cu), Bor (B), Kalzium (Ca), Zink (Zn), Zirkonium (Zr), Niob (Nb), Molybdän (Mo), Strontium (Sr), Antimon (Sb), Wolfram (W) und Wismut (Bi); und
A ein Element ist, das eine Oxidationszahl von -1 oder -2 aufweist; 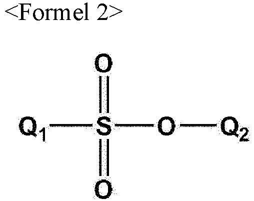
wobei Q₁ und Q₂ in Formel 2 jeweils unabhängig eine substituierte oder unsubstituierte C₁-C₂₀-Alkylgruppe sind,
wobei eine Menge der Alkylsulfonatverbindung in einem Bereich von 0,005 Gewichtsteilen bis 5 Gewichtsteilen, bezogen auf 100 Gewichtsteile des Elektrolyten, ist, und
wobei substituiert bedeutet, dass mindestens ein Wasserstoffatom substituiert ist durch ein Halogenatom, eine C₁-C₂₀-Alkylgruppe substituiert durch ein Halogenatom, eine C₁-C₂₀-Alkoxygruppe, eine C₂-C₂₀-Alkoxyalkylgruppe, eine Hydroxylgruppe, eine Nitrogruppe, eine Cyanogruppe, eine Aminogruppe, eine Amidinogruppe, Hydrazin, Hydrazon, eine Carboxylgruppe oder ein Salz davon, eine Sulfonylgruppe, eine Sulfamoylgruppe, Sulfonsäuregruppe oder ein Salz davon, Phosphorsäure oder ein Salz davon, eine C₁-C₂₀-Alkylgruppe, eine C₂-C₂₀-Alkenylgruppe, eine C₂-C₂₀-Alkinylgruppe, eine C₁-C₂₀-Heteroalkylgruppe, eine C₆-C₂₀-Arylgruppe, eine C₆-C₂₀-Arylalkylgruppe, eine C₆-C₂₀-Heteroarylgruppe, eine C₇-C₂₀-Heteroarylakylgruppe, eine C₆-C₂₀-Heteroaryloxygruppe, eine C₆-C₂₀-Heteroaryloxyalkylgruppe oder eine C₆-C₂₀-Heteroarylalkylgruppe.

2. Lithiumbatterie nach Anspruch 1, wobei Q₁ und Q₂ jeweils unabhängig ausgewählt sind aus: einer Methylgruppe, einer Ethylgruppe, einer Propylgruppe, einer Isopropylgruppe, einer Butylgruppe, einer sec-Butylgruppe, einer tert-Butylgruppe und einer Isobutylgruppe, und
einer Methylgruppe, einer Ethylgruppe, einer Propylgruppe, einer Isopropylgruppe, einer Butylgruppe, einer sec-Butylgruppe, einer tert-Butylgruppe oder einer Isobutylgruppe, jeweils substituiert mit mindestens einer Gruppe, ausgewählt aus Deuterium, -F, -Cl, -Br, -I, einer Cyanogruppe, einer Nitrogruppe, einer Hydroxylgruppe, einer Methylgruppe, einer Ethylgruppe und einer Propylgruppe.

3. Lithiumbatterie nach Anspruch 1 oder 2, wobei die Alkylsulfonatverbindung ausgewählt ist aus den Verbindungen 1 und 2 unten: 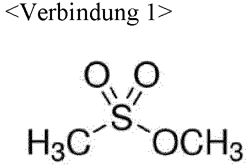 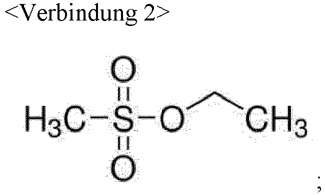 und/oder
wobei das Lithiumsalz mindestens eines umfasst, ausgewählt aus LiPF₆, LiBF₄, LiCF₃SO₃, Li(CF₃SO₂)₂N, LiC₂F₅SO₃, Li(FSO₂)₂N, LiC₄F₉SO₃, LiN(SO₂CF₂CF₃)₂, und Verbindungen, dargestellt durch Formel 22 bis 25 unten: 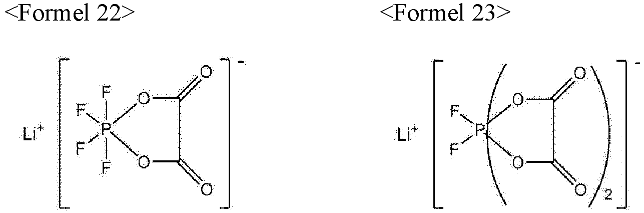 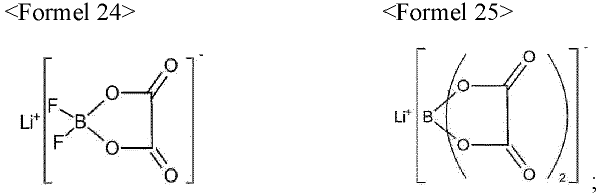 und/oder
wobei das nicht-wässrige Lösungsmittel ausgewählt ist aus Dimethylcarbonat (DMC), Diethylcarbonat (DEC), Ethylmethylcarbonat (EMC), Dipropylcarbonat (DPC), Methylpropylcarbonat (MPC), Ethylpropylcarbonat (EPC), Methylethylcarbonat (MEC), Ethylencarbonat (EC), Propylencarbonat (PC), Butylencarbonat (BC), Methylpropionat (MP), Ethylpropionat, Propylpropionat (PP), Tetraethylenglykoldimethylether (TEGDME) und Gemischen davon.

4. Lithiumbatterie nach einem der Ansprüche 1-3, wobei der Elektrolyt ferner eines umfasst, ausgewählt aus Fluorethylencarbonat (FEC), Vinylencarbonat (VC), Vinylethylencarbonat (VEC), Maleinsäureanhydrid, Bernsteinsäureanhydrid, einer phosphorhaltigen (P) Verbindung, einer schwefelhaltigen (S) Verbindung und Gemischen davon,
wobei die P-haltige Verbindung mindestens eine umfasst, ausgewählt aus einer Phosphinverbindung, einer Phosphatverbindung und einer Phosphitverbindung, und
die S-haltige Verbindung mindestens eine umfasst, ausgewählt aus einer Sulfonverbindung, einer Sulfonatverbindung, einer Sultonverbindung und einer Disulfonatverbindung.

5. Lithiumbatterie nach einem der Ansprüche 1-4, wobei das aktive positive Elektrodenmaterial dargestellt ist durch Formel 10 oder 20:
<Formel 10> Li_{x'}Ni_{y'}Co_{1-y'-z'}Al_{z'}O₂
<Formel 20> Li_{x'}Ni_{y'}Co_{1-y'-z'}Mn_{z'}O₂
wobei in Formel 10 und 20 0,9≤x'≤1,2, 0,88≤y'≤0,98, 0<z'<0,1, und 0<1- y'-z'<0,2 erfüllt sind; und/oder
wobei die positive Elektrode mindestens eines umfasst, ausgewählt aus Li_{1,02}Ni_{0,88}Co_{0,08}Mn_{0,04}O₂, Li_{1,02}Ni_{0,88}Co_{0,10}Mn_{0,02}O₂, Li_{1,02}Ni_{0,91}Co_{0,06}Mn_{0,03}O₂,
LiNi_{0,94}Co_{0,04}Mn_{0,02}O₂, Li_{1,02}Ni_{0,88}Co_{0,08}Al_{0,04}O₂, Li_{1,02}Ni_{0,88}Co_{0,10}Al_{0,02}O₂,
Li_{1,02}Ni_{0,91}Co_{0,06}Al_{0,03}O₂ und LiNi_{0,94}Co_{0,04}Al_{0,02}O₂; und/oder
wobei die negative Elektrode ein aktives negatives Elektrodenmaterial umfasst, wobei das aktive negative Elektrodenmaterial mindestens eines umfasst, ausgewählt aus einer Verbindung auf Siliziumbasis, einer Verbindung auf Kohlenstoffbasis, einem Verbundstoff aus einer Verbindung auf Siliziumbasis und einer Verbindung auf Kohlenstoffbasis und einem Siliziumoxid (SiOₓ₁, wobei 0<x1<2).

6. Lithiumbatterie nach einem der Ansprüche 1-5, wobei der Elektrolyt ferner eine ungesättigte Sulfonverbindung beinhaltet, dargestellt durch Formel 3 unten: 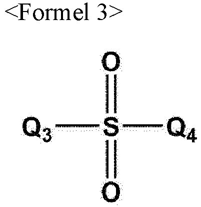
wobei Q₃ und Q₄ in Formel 3 jeweils unabhängig ausgewählt sind aus einer Gruppe, dargestellt durch -(L₁)-(R₁), eine Vinylgruppe, eine Allylgruppe und eine substituierte oder unsubstituierte C₁-C₂₀-Alkylgruppe,
mindestens eines von Q₃ und Q₄ eine Gruppe ist, dargestellt durch -(L₁)-(R₁), eine Vinylgruppe oder eine Allylgruppe,
L₁ ausgewählt ist aus einer substituierten oder unsubstituierten C₂-C₂₀-Alkenylengruppe und einer substituierten oder unsubstituierten C₂-C₂₀-Alkinylengruppe, und
R₁ ausgewählt ist aus Wasserstoff und einer substituierten oder unsubstituierten C₂-C₂₀-Alkylgruppe,
wobei substituiert bedeutet, dass mindestens ein Wasserstoffatom substituiert ist durch ein Halogenatom, eine C₁-C₂₀-Alkylgruppe substituiert durch ein Halogenatom, eine C₁-C₂₀-Alkoxygruppe, eine C₂-C₂₀-Alkoxyalkylgruppe, eine Hydroxylgruppe, eine Nitrogruppe, eine Cyanogruppe, eine Aminogruppe, eine Amidinogruppe, Hydrazin, Hydrazon, eine Carboxylgruppe oder ein Salz davon, eine Sulfonylgruppe, eine Sulfamoylgruppe, Sulfonsäuregruppe oder ein Salz davon, Phosphorsäure oder ein Salz davon, eine C₁-C₂₀-Alkylgruppe, eine C₂-C₂₀-Alkenylgruppe, eine C₂-C₂₀-Alkinylgruppe, eine C₁-C₂₀-Heteroalkylgruppe, eine C₆-C₂₀-Arylgruppe, eine C₆-C₂₀-Arylalkylgruppe, eine C₆-C₂₀-Heteroarylgruppe, eine C₇-C₂₀-Heteroarylakylgruppe, eine C₆-C₂₀-Heteroaryloxygruppe, eine C₆-C₂₀-Heteroaryloxyalkylgruppe oder eine C₆-C₂₀-Heteroarylalkylgruppe.

7. Lithiumbatterie nach Anspruch 6, wobei die Menge der Alkylsulfonatverbindung in einem Bereich von 0,001 Gewichtsteilen bis 3 Gewichtsteilen, bezogen auf 100 Gewichtsteile des Elektrolyten, ist; und/oder
wobei eine Menge der ungesättigten Sulfonverbindung in einem Bereich von 0,001 Gewichtsteilen bis 3 Gewichtsteilen, bezogen auf 100 Gewichtsteile des Elektrolyten, ist.

8. Lithiumbatterie nach Anspruch 6 oder 7, wobei Q₁ und Q₂ jeweils unabhängig ausgewählt sind aus: einer Methylgruppe, einer Ethylgruppe, einer Propylgruppe, einer Isopropylgruppe, einer Butylgruppe, einer sec-Butylgruppe, einer tert-Butylgruppe und einer Isobutylgruppe; und
einer Methylgruppe, einer Ethylgruppe, einer Propylgruppe, einer Isopropylgruppe, einer Butylgruppe, einer sec-Butylgruppe, einer tert-Butylgruppe oder einer Isobutylgruppe, jeweils substituiert mit mindestens einer Gruppe, ausgewählt aus Deuterium, -F, -Cl, -Br, **-I,** einer Cyanogruppe, einer Nitrogruppe, einer Hydroxylgruppe, einer Methylgruppe, einer Ethylgruppe und einer Propylgruppe, und
Q₃ und Q₄ jeweils unabhängig ausgewählt sind aus: einer Vinylgruppe, einer Allylgruppe, einer Methylgruppe, einer Ethylgruppe, einer Propylgruppe, einer Isopropylgruppe, einer Butylgruppe, einer sec-Butylgruppe, einer tert-Butylgruppe und einer Isobutylgruppe; und
einer Methylgruppe, einer Ethylgruppe, einer Propylgruppe, einer Isopropylgruppe, einer Butylgruppe, einer sec-Butylgruppe, einer tert-Butylgruppe oder einer Isobutylgruppe, jeweils substituiert mit mindestens einer Gruppe, ausgewählt aus Deuterium, -F, -Cl, -Br, **-I,** einer Cyanogruppe, einer Nitrogruppe, einer Hydroxylgruppe, einer Methylgruppe, einer Ethylgruppe und einer Propylgruppe, und
mindestens eines von Q₃ und Q₄ eine Vinylgruppe oder eine Allylgruppe ist.

9. Lithiumbatterie nach einem der Ansprüche 6-8, wobei die Alkylsulfonatverbindung ausgewählt ist aus Verbindung 1 und 2 unten: 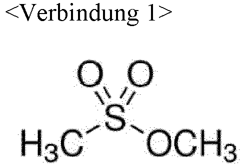 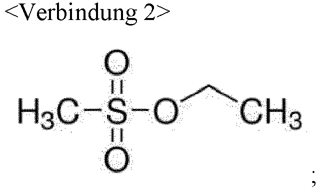 und/oder
wobei die ungesättigte Sulfonverbindung Verbindung 3 unten ist:

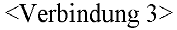

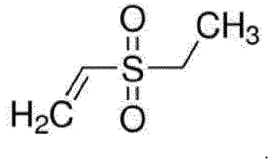

10. Lithiumbatterie nach einem der Ansprüche 6-9, wobei der Elektrolyt keine aromatische Kohlenwasserstoffverbindung oder eine mit einem Halogen substituierte aromatische Kohlenwasserstoffverbindung umfasst.

11. Lithiumbatterie nach einem der Ansprüche 6-10, wobei die Konzentration des Lithiumsalzes in dem Elektrolyten in einem Bereich von 1,0 M bis 1,5 M ist.

12. Lithiumbatterie nach einem der Ansprüche 6-11, wobei der Elektrolyt ferner eines umfasst, ausgewählt aus Fluorethylencarbonat (FEC), Vinylencarbonat (VC), Vinylethylencarbonat (VEC), Maleinsäureanhydrid, Bernsteinsäureanhydrid, einer phosphorhaltigen (P) Verbindung, einer schwefelhaltigen (S) Verbindung und Gemischen davon,
wobei die P-haltige Verbindung mindestens eine umfasst, ausgewählt aus einer Phosphinverbindung, einer Phosphatverbindung und einer Phosphitverbindung, und
die S-haltige Verbindung mindestens eine umfasst, ausgewählt aus einer Sulfonverbindung, einer Sulfonatverbindung, einer Sultonverbindung und einer Disulfonatverbindung;
und wobei die Menge von FEC, VC, VEC, Maleinsäureanhydrid und Bernsteinsäureanhydrid jeweils in einem Bereich von 0,1 Gewichtsteilen bis 2 Gewichtsteilen, bezogen auf 100 Gewichtsteile des Elektrolyten, ist.

## Revendications

1. Batterie au lithium comprenant : une électrode positive ; une électrode négative ; et un électrolyte entre l'électrode positive et l'électrode négative,
dans laquelle l'électrode positive comprend un matériau actif d'électrode positive représenté par la formule 1A ci-dessous, et
l'électrolyte comprend un sel de lithium, un solvant non aqueux et un composé alkylsulfonate représenté par la formule 2 ci-dessous :
<Formule 1A> LiₓNi_{y}M_{1-y}O_{2-z}A_{z}
dans laquelle, dans la formule 1A, 0,9≤x≤1,2, 0,88≤y≤0,98 et 0≤z<0,2 sont satisfaites,
M est au moins un élément choisi dans le groupe constitué par l'aluminium (Al), le magnésium (Mg), le manganèse (Mn), le cobalt (Co), le fer (Fe), le chrome (Cr), le vanadium (V), le titane (Ti), le cuivre (Cu), le bore (B), le calcium (Ca), le zinc (Zn), le zirconium (Zr), le niobium (Nb), le molybdène (Mo), le strontium (Sr), l'antimoine (Sb), le tungstène (W) et le bismuth (Bi) ; et
A est un élément comportant un nombre d'oxydation de -1 ou -2 ; 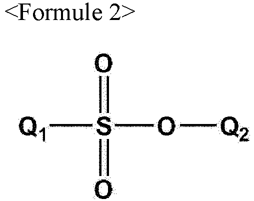
dans laquelle, dans la formule 2, Q₁ et Q₂ sont chacun indépendamment un groupe alkyle en C₁ à C₂₀ substitué ou non substitué,
dans laquelle une quantité du composé alkylsulfonate est comprise dans une plage de 0,005 partie en poids à 5 parties en poids par rapport à 100 parties en poids de l'électrolyte, et
dans laquelle substitué signifie qu'au moins un atome d'hydrogène est substitué par un atome d'halogène, un groupe alkyle en C₁ à C₂₀ substitué par un atome d'halogène, un groupe alcoxy en C₁ à C₂₀, un groupe alcoxyalkyle en C₂ à C₂₀, un groupe hydroxyle, un groupe nitro, un groupe cyano, un groupe amino, un groupe amidino, l'hydrazine, l'hydrazone, un groupe carboxyle ou un sel de celui-ci, un groupe sulfonyle, un groupe sulfamoyle, un groupe acide sulfonique ou un sel de celui-ci, l'acide phosphorique ou un sel de celui-ci, un groupe alkyle en C₁ à C₂₀, un groupe alcényle en C₂ à C₂₀, un groupe alcynyle en C₂ à C₂₀, un groupe hétéroalkyle en C₁ à C₂₀, un groupe aryle en C₆ à C₂₀, un groupe arylalkyle en C₆ à C₂₀, un groupe hétéroaryle en C₆ à C₂₀, un groupe hétéroarylakyle en C₇ à C₂₀, un groupe hétéroaryloxy C₆ à C₂₀, un groupe hétéroaryloxyalkyle en C₆ à C₂₀, ou un groupe hétéroarylalkyle en C₆ à C₂₀.

2. Batterie au lithium selon la revendication 1, dans laquelle Q₁ et Q₂ sont chacun indépendamment choisis parmi : un groupe méthyle, un groupe éthyle, un groupe propyle, un groupe isopropyle, un groupe butyle, un groupe sec-butyle, un groupe tert-butyle et un groupe isobutyle, et
un groupe méthyle, un groupe éthyle, un groupe propyle, un groupe isopropyle, un groupe butyle, un groupe sec-butyle, un groupe tert-butyle ou un groupe isobutyle, chacun substitué par au moins un choisi parmi le deutérium, -F, -Cl, -Br, -I, un groupe cyano, un groupe nitro, un groupe hydroxyle, un groupe méthyle, un groupe éthyle et un groupe propyle.

3. Batterie au lithium selon les revendications 1 ou 2, dans laquelle le composé alkylsulfonate est choisi parmi les composés 1 et 2 ci-dessous : 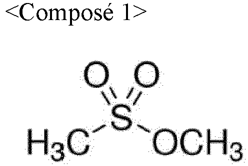 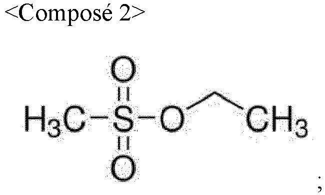 et/ou
dans laquelle le sel de lithium comprend au moins un choisi parmi LiPF₆, LiBF₄, LiCF₃SO₃, Li(CF₃SO₂)₂N, LiC₂F₅SO₃, Li(FSO₂)₂N, LiC₄F₉SO₃, LiN(SO₂CF₂CF₃)₂, et des composés représentés par les formules 22 à 25 ci-dessous : 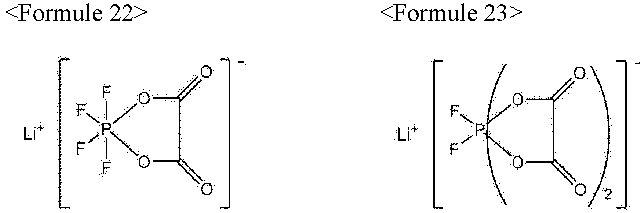 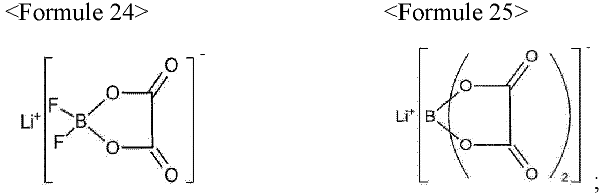 et/ou
dans laquelle le solvant non aqueux est choisi parmi le carbonate de diméthyle (DMC), le carbonate de diéthyle (DEC), le carbonate d'éthyle et de méthyle (EMC), le carbonate de dipropyle (DPC), le carbonate de méthyle et de propyle (MPC), le carbonate d'éthyle et de propyle (EPC), le carbonate de méthyle et d'éthyle (MEC), le carbonate d'éthylène (EC), le carbonate de propylène (PC), le carbonate de butylène (BC), le propionate de méthyle (MP), le propionate d'éthyle, le propionate de propyle (PP), l'éther diméthylique de tétraéthylène glycol (TEGDME) et leurs mélanges.

4. Batterie au lithium selon l'une quelconque des revendications 1 à 3, dans laquelle l'électrolyte comprend en outre un choisi parmi le carbonate de fluoroéthylène (FEC), le carbonate de vinylène (VC), le carbonate de vinyle éthylène (VEC), l'anhydride maléique, l'anhydride succinique, un composé contenant du phosphore (P), un composé contenant du soufre (S) et leurs mélanges,
dans laquelle le composé contenant du P comprend au moins un choisi parmi un composé de phosphine, un composé de phosphate et un composé de phosphite, et
le composé contenant du S comprend au moins un choisi parmi un composé sulfone, un composé sulfonate, un composé sultone et un composé disulfonate.

5. Batterie au lithium selon l'une quelconque des revendications 1 à 4, dans laquelle le matériau actif d'électrode positive est représenté par la formule 10 ou 20 :
<Formule 10> Li_{x'}Ni_{y'}Co_{1-y'-z'}Al_{z'}O₂
<Formule 20> Li_{x'}Ni_{y'}Co_{1-y'-z'}Mn_{z'}O₂
dans laquelle, dans les formules 10 et 20, 0,9≤x'≤1,2, 0,88≤y'≤0,98, 0<z'<0,1 et 0<1-y'-z'<0,2 sont satisfaites ; et/ou
dans laquelle l'électrode positive comprend au moins un choisi parmi Li_{1,02}N_{10,88}Co_{0,08}Mn_{0,04}O₂, Li_{1,02}Ni_{0,88}Co_{0,10}Mn_{0,02}O₂, Li_{1,02}Ni_{0,91}Co_{0,06}Mn_{0,03}O₂, LiNi_{0,94}Co_{0,04}Mn_{0,02}O₂, Li_{1,02}Ni_{0,88}Co_{0,08}Al_{0,0}4O₂, Li_{1,02}Ni_{0,88}Co_{0,10}Al_{0,02}O₂, Li_{1,02}Ni_{0,91}Co_{0,06}Al_{0,03}O₂, et LiNi_{0,94}Co_{0,04}Al_{0,02}O₂; et/ou
dans laquelle l'électrode négative comprend un matériau actif d'électrode négative, le matériau actif d'électrode négative comprenant au moins un choisi parmi un composé à base de silicium, un composé à base de carbone, un composite d'un composé à base de silicium et d'un composé à base de carbone, et un oxyde de silicium (SiOₓ₁ où 0<x1<2).

6. Batterie au lithium selon l'une quelconque des revendications 1 à 5, dans laquelle l'électrolyte comprend en outre un composé sulfone insaturé représenté par la formule 3 ci-dessous : 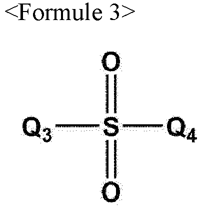
dans laquelle, dans la formule 3, Q₃ et Q₄ sont chacun indépendamment choisis parmi un groupe représenté par -(L₁)-(R₁), un groupe vinyle, un groupe allyle et un groupe alkyle en C₁ à C₂₀ substitué ou non substitué,
au moins l'un de Q₃ et Q₄ est un groupe représenté par -(L₁)-(R₁), un groupe vinyle ou un groupe allyle,
L₁ est choisi parmi un groupe alcénylène en C₂ à C₂₀ substitué ou non substitué et un groupe alcynylène en C₂ à C₂₀ substitué ou non substitué, et
R₁ est choisi parmi l'hydrogène et un groupe alkyle en C₂ à C₂₀ substitué ou non substitué,
dans laquelle substitué signifie qu'au moins un atome d'hydrogène est substitué par un atome d'halogène, un groupe alkyle en C₁ à C₂₀ substitué par un atome d'halogène, un groupe alcoxy en C₁ à C₂₀, un groupe alcoxyalkyle en C₂ à C₂₀, un groupe hydroxyle, un groupe nitro, un groupe cyano, un groupe amino, un groupe amidino, l'hydrazine, l'hydrazone, un groupe carboxyle ou un sel de celui-ci, un groupe sulfonyle, un groupe sulfamoyle, un groupe acide sulfonique ou un sel de celui-ci, l'acide phosphorique ou un sel de celui-ci, un groupe alkyle en C₁ à C₂₀, un groupe alcényle en C₂ à C₂₀, un groupe alcynyle en C₂ à C₂₀, un groupe hétéroalkyle en C₁ à C₂₀, un groupe aryle en C₆ à C₂₀, un groupe arylalkyle en C₆ à C₂₀, un groupe hétéroaryle en C₆ à C₂₀, un groupe hétéroarylakyle en C₇ à C₂₀, un groupe hétéroaryloxy en C₆ à C₂₀, un groupe hétéroaryloxyalkyle en C₆ à C₂₀ ou un groupe hétéroarylalkyle en C₆ à C₂₀.

7. Batterie au lithium selon la revendication 6, dans laquelle une quantité du composé alkylsulfonate est comprise dans une plage de 0,001 partie en poids à 3 parties en poids par rapport à 100 parties en poids de l'électrolyte ; et/ou
dans laquelle une quantité du composé sulfone insaturé est comprise dans une plage de 0,001 partie en poids à 3 parties en poids par rapport à 100 parties en poids de l'électrolyte.

8. Batterie au lithium selon les revendications 6 ou 7, dans laquelle Q₁ et Q₂ sont chacun indépendamment choisis parmi : un groupe méthyle, un groupe éthyle, un groupe propyle, un groupe isopropyle, un groupe butyle, un groupe sec-butyle, un groupe tert-butyle et un groupe isobutyle ; et
un groupe méthyle, un groupe éthyle, un groupe propyle, un groupe isopropyle, un groupe butyle, un groupe sec-butyle, un groupe tert-butyle ou un groupe isobutyle, chacun substitué par au moins un choisi parmi le deutérium, -F, -Cl, -Br, **-I,** un groupe cyano, un groupe nitro, un groupe hydroxyle, un groupe méthyle, un groupe éthyle et un groupe propyle, et
Q₃ et Q₄ sont chacun indépendamment sélectionnés parmi : un groupe vinyle, un groupe allyle, un groupe méthyle, un groupe éthyle, un groupe propyle, un groupe isopropyle, un groupe butyle, un groupe sec-butyle, un groupe tert-butyle et un groupe isobutyle ; et
un groupe méthyle, un groupe éthyle, un groupe propyle, un groupe isopropyle, un groupe butyle, un groupe sec-butyle, un groupe tert-butyle ou un groupe isobutyle, chacun substitué par au moins un choisi parmi le deutérium, -F, -Cl, -Br, **-I,** un groupe cyano, un groupe nitro, un groupe hydroxyle, un groupe méthyle, un groupe éthyle et un groupe propyle, et
au moins l'un de Q₃ et Q₄ est un groupe vinyle ou un groupe allyle.

9. Batterie au lithium selon l'une quelconque des revendications 6 à 8, dans laquelle le composé alkylsulfonate est choisi parmi les composés 1 et 2 ci-dessous : 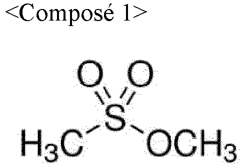 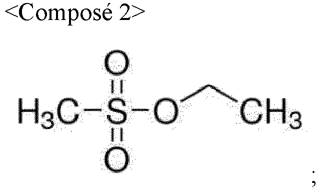 et/ou
dans laquelle le composé sulfone insaturé est le composé 3 ci-dessous :

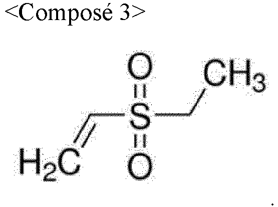

10. Batterie au lithium selon l'une quelconque des revendications 6 à 9, dans laquelle l'électrolyte ne comprend pas de composé hydrocarboné aromatique ou de composé hydrocarboné aromatique substitué par un halogène.

11. Batterie au lithium selon l'une quelconque des revendications 6 à 10, dans laquelle une concentration du sel de lithium dans l'électrolyte est comprise dans une plage de 1,0 M à 1,5 M.

12. Batterie au lithium selon l'une quelconque des revendications 6 à 11, dans laquelle l'électrolyte comprend en outre un composé choisi parmi le carbonate de fluoroéthylène (FEC), le carbonate de vinylène (VC), le carbonate de vinyle éthylène (VEC), l'anhydride maléique, l'anhydride succinique, un composé contenant du phosphore (P), un composé contenant du soufre (S) et leurs mélanges,
dans laquelle le composé contenant du P comprend au moins un choisi parmi un composé de phosphine, un composé de phosphate et un composé de phosphite, et
le composé contenant du S comprend au moins un choisi parmi un composé sulfone, un composé sulfonate, un composé sultone et un composé disulfonate ;
et dans laquelle une quantité de chacun des FEC, VC, VEC, anhydride maléique et anhydride succinique est comprise dans une plage de 0,1 partie en poids à 2 parties en poids par rapport à 100 parties en poids de l'électrolyte.
